# EUROPEAN PATENT APPLICATION

(11) **EP 4 570 907 A1**
(43) Date of publication of application: **18.06.2025**
(21) Application number: 23852584.4
(22) Date of filing: 08.08.2023
(51) Int. Cl.: C12N 15/11, C07K 4/12, C07K 14/435, C12M 1/00, C12Q 1/6869

(54) **METHOD FOR SELECTING SUBJECT-DERIVED NEOANTIGEN**

(30) Priority: 08.08.2022 JP 2022126148
(71) Applicant: Sapporo Medical University, Sapporo-shi, Hokkaido 060-8556 (JP)
(72) Inventor: KANASEKI, Takayuki, Sapporo-shi, Hokkaido 060-8556 (JP); TOKITA, Serina, Sapporo-shi, Hokkaido 060-8556 (JP); TORIGOE, Toshihiko, Sapporo-shi, Hokkaido 060-8556 (JP)
(74) Representative: Meissner Bolte Partnerschaft mbB
(86) International application number: PCT/JP2023/029006
(87) International publication number: WO 2024/034622

(57) **Abstract**

The purpose of the present invention is to provide a means for selecting subject-derived neoantigens. The above problem is solved by providing a method for selecting a subject-derived neoangiten, said method comprising: a step of acquiring sequence data of a normal cell and a cancer cell derived from a subject, a step of identifying a gene having a genetic mutation specific to the cancer cell, and a step of identifying a peptide based on a wild-type gene corresponding to the gene having the genetic mutation specific to the cancer cell from a major histocompatibility complex (MHC)-presented peptide database, wherein the genetic mutation is a missense mutation, and the peptide based on the wild-type gene corresponding to the gene having the genetic mutation has a wild-type amino acid corresponding to the position of an amino acid mutation due to the genetic mutation.

## Description

### [Technical Field]

The invention relates to methods for selecting subject-derived neoantigens.

### [Background Technology]

Cancer treatment methods include surgery, radiation therapy, and drug therapy, as well as immunotherapy. Immunotherapy is a treatment method utilizing the immune response against cancer that originally exists in the living body, and it controls the growth of cancer by enhancing such immune response. Immunotherapy is expected to be a treatment method that acts on the entire organism while providing long-lasting therapeutic effects with few side effects.

Immune cells responsible for immunotherapy include T cells, especially CD8-positive T cells (cytotoxic T cells: CTLs) and CD4-positive T cells. Cancer cells present peptides derived from specific proteins, i.e., cancer antigens, that are highly expressed in cancer cells compared to normal cells, to CTLs via major histocompatibility complex (MHC) class I molecules on the surface of the cancer cells. CTLs recognize and activate such peptides via T cell receptors (TCRs), thereby eliminating the cancer cells. In other words, by inducing CTLs to recognize such peptides, the immune response against cancer can be enhanced and immunotherapy can be realized. In addition, CD4-positive T cells promote the immune response by CTLs by recognizing and activating peptides presented via MHC class II molecules. That is, by inducing CD4-positive T cells to recognize such peptides, the immune response against cancer can also be enhanced and immunotherapy cab be realized.

Recently, it has been reported that, in addition to the above-mentioned peptides derived from cancer antigens, peptides derived from specific proteins having mutations that exist specifically only in cancer cells but not in normal cells (said peptides have mutations that specifically exist only in cancer cells) can also be used for immunotherapy as cancer cell-derived peptides (non-patent literature 1). Such specific protein-derived peptides are called new cancer cell-derived antigens, or "neoantigens". Genetic mutations that result in neoantigens are only marginally present in the vast number of genetic mutations in cancer cells. Since such genetic mutations are different in each cancer patient, neoantigens are also different in each cancer patient.

Cancer cells present neoantigens to CD8-positive T cells and CD4-positive T cells via MHC class I and MHC class II molecules on the surface of the cancer cells. CD8-positive T cells and CD4-positive T cells recognize and activate neoantigens via TCRs, thereby distinguishing between normal cells and cancer cells and specifically eliminating cancer cells. In other words, by inducing CD8-positive T cells and CD4-positive T cells to recognize neoantigens via TCRs, the immune response against cancer can be specifically enhanced and highly specific immunotherapy can be achieved. In particular, since neoantigens are different in each cancer patient, by inducing CD8-positive T cells and CD4-positive T cells to recognize neoantigens via TCRs, the immune response against cancer can be specifically enhanced in each cancer patient, and highly specific immunotherapy personalized for each patient can be realized.

In addition, since neoantigens have mutations that are not present in normal cells but only specifically in cancer cells, and they do not originally exist in the living body, immune tolerance in the thymus can be avoided. Thus, it is expected that a strong immune response can be induced by inducing CD8-positive T cells and CD4-positive T cells to recognize neoantigens via TCRs.

Methods for predicting neoantigens that differ for each cancer patient include in silico method and mass spectrometry (MS) method. The in silico method uses recent highly developed next-generation sequencing technology to identify cancer cell-specific genetic mutations in each cancer patient, and then uses an MHC-binding prediction algorithm to predict peptides that have the genetic mutation and that can be presented to the MHC, thereby predicting neoantigens that differ for each cancer patient (non-patent literature 2). This method has the advantage of high sensitivity and simplicity because it uses the above-mentioned next-generation sequencing technologies and algorithms, but it has a problem with specificity because whether the predicted neoantigens are actually presented to the MHC in the living body is unknown. The MS method predicts different neoantigens for each cancer patient by enriching MHC-peptide complexes from cancer cells for each cancer patient, eluting peptides from the MHC of the complexes, and then identifying the peptides by MS (non-patent literature 3). This method has the advantage of high specificity because the peptides actually presented to the MHC in the living body are identified by MS; however, the peptides are limited to those that are abundant in the living body, and this method involves complicated experimental manipulations, resulting in problems in sensitivity and simplicity.

### [Prior Art Literatures]

### [Non-patent Literatures]

[Non-patent literature 1] Nat. Biotechnol. 35, 97 (2017).
[Non-patent literature 2] Nat. Rev. Drug Discov. 21, 261-282 (2022).
[Non-patent literature 3] Nat. Rev. Clin. Oncol. 17, 595-610 (2020).

### [Summary of Invention]

### [Problems to Be Solved by Invention]

The purpose of the present invention is to provide a means for selecting subject-derived neoantigens.

### [Means for Solving Problems]

In the course of researching methods for selecting subject-derived neoantigens, the inventors have focused on the possibility of combining conventional in silico method and MS method to simultaneously obtain advantages of the former, high sensitivity and simplicity, and the latter, high specificity. As a result of intensive research to verify such a possibility, the inventors have found that subject-derived neoantigens can be selected by identifying genes having genetic mutations specific to cancer cells by mutually comparing sequence data of normal cells and cancer cells, and by identifying peptides based on wild-type genes corresponding to said genes from an MHC-presented peptide database; based on this finding, the inventors have conducted further research and completed the present invention.

For certain cancers, it is extremely difficult to collect a sufficient amount of tumor biopsies from an organ and to isolate the cells. The inventors found that neoantigens can be selected by using an MHC-presented peptide database obtained from cells other than the subject's own cancer cells. In the present specification, such a database is sometimes referred to as a surrogate immunopeptidome. That is, the origin of the cells used for producing the surrogate immunopeptidome does not depend on the organ or tissue, nor does it depend on whether the cells are derived from the subject patient or a third party. In addition, blood-derived cells can be used in the surrogate immunopeptidome. Cells derived from blood are generally easy and rapid to collect and isolate. Even if sufficient blood cells cannot be obtained, a sufficient amount of cells can be secured for MS analysis by growing them using EBV. The inventors have made it possible to identify neoantigen peptides using the surrogate immunopeptidome. For example, it has become possible to select neoantigens derived from a subject by identifying genes having genetic mutations specific to cancer cells by mutually comparing sequence data of cells collected from the subject's cancer biopsy tissue and non-cancerous tissue, and identifying peptides based on wild-type genes corresponding to the identified genes from the surrogate immunopeptidome.

That is, the present invention relates to the followings:
[1] A method for selecting a neoantigen derived from a subject, comprising: (1) a step of acquiring sequence data of a normal cell and a cancer cell derived from the subject, (2) a step of identifying a gene having a genetic mutation specific to the cancer cell by mutually comparing the sequence data of the normal cell and the cancer cell, and (3) a step of identifying a peptide based on a wild-type gene corresponding to the gene having the genetic mutation specific to the cancer cell from a major histocompatibility complex (MHC)-presented peptide database, wherein the genetic mutation specific to the cancer cell is a missense mutation, and the peptide based on the wild-type gene corresponding to the gene having the genetic mutation specific to the cancer cell has a wild-type amino acid corresponding to the position of an amino acid mutation due to the genetic mutation.
[2] The method according to [1], wherein the sequence data is whole exome sequence data.
[3] The method according to [2], wherein the MHC-presented peptide database comprises a list of wild-type MHC-presented peptides for each MHC class and/or each organ type.
[4] The method according to [1], wherein the MHC-presented peptide database comprises a list of wild-type MHC-presented peptides.
[5] The method according to [4], wherein step (3) is a step of identifying a peptide based on a wild-type gene corresponding to the gene having the genetic mutation specific to the cancer cell, from the MHC-presented peptide database for each MHC class and/or each organ type.
[6] The method according to [5], wherein the amino acid mutation due to the genetic mutation is a single amino acid mutation.
[7] The method according to [6], wherein the peptide based on the wild-type gene corresponding to the gene having the genetic mutation specific to the cancer cell is scored based on its MHC presentation frequency in the MHC-presented peptide database.
[8] The method according to [7], wherein the peptide based on the wild-type gene corresponding to the gene having the genetic mutation specific to the cancer cell is identified based on the scoring.
[9] The method according to [8], wherein the MHC-presented peptide database is created by an MHC-presented peptide analysis method comprising a step of obtaining an MHC-peptide complex from a cell, a step of obtaining a peptide from said complex, and a step of identifying the amino acid sequence of said peptide by mass spectrometry.
[10] The method according to [9], wherein the cell is not from a subject or a non-subject.
[11] The method according to [9], wherein the cell is collected from blood.
[12] The method according to [9], wherein the MHC is MHC class I and/or class II.
[13] The method according to [12], wherein the MHC is a human leukocyte antigen (HLA).
[14] The method according to [13], further comprising, in the peptide based on the wild-type gene corresponding to the gene having the genetic mutation specific to the cancer cell, said peptide having a wild-type amino acid corresponding to the position of an amino acid mutation due to the genetic mutation, a step of introducing an amino acid mutation due to the genetic mutation and/or a step of determining the gene sequence encoding the peptide into which the amino acid mutation has been introduced.
[15] The method according to [14], further comprising a step of selecting the peptide into which the amino acid mutation has been introduced and/or the nucleic acid consisting of the base sequence into which the genetic mutation has been introduced as a neoantigen derived from the subject.
[16] A method for producing a peptide containing a neoantigen derived from a subject, comprising a step of adding 0 or more optional amino acids to the N-terminus and/or C-terminus of the neoantigen derived from the subject selected by the method according to [15], wherein the number of amino acids contained in the neoantigen to which the amino acids have been added is 40 or less.
[17] A peptide obtained by the method according to [16].
[18] A method for producing a nucleic acid containing a neoantigen derived from a subject, comprising a step of adding 0 or more optional bases to the 5' end and/or 3' end of a nucleic acid containing the neoantigen derived from the subject selected by the method according to [15].
[19] A nucleic acid containing a neoantigen derived from a subject, wherein the neoantigen is selected by the method according to [15].
[20] The method according to [18], further comprising a step of producing an mRNA containing a nucleic acid containing a neoantigen derived from the subject.
[21] An mRNA containing a neoantigen derived from a subject, wherein the neoantigen is selected by the method according to [15].
[22] A vaccine composition comprising the peptide according to [17], the nucleic acid according to [19], or the mRNA according to [21].
[23] A system for selecting a neoantigen derived from a subject, comprising: (1) a sequence data acquisition section to acquire sequence data of a normal cell and a cancer cell derived from the subject, (2) a gene identification section to identify a gene having a genetic mutation specific to the cancer cell by mutually comparing the sequence data of the normal cell and the cancer cell, and (3) a peptide identification section to identify a peptide based on a wild-type gene corresponding to the gene having the genetic mutation specific to the cancer cell from a major histocompatibility complex (MHC)-presented peptide database, wherein the genetic mutation specific to the cancer cell is a missense mutation, and the peptide based on the wild-type gene corresponding to the gene having the genetic mutation specific to the cancer cell has a wild-type amino acid corresponding to the position of an amino acid mutation due to the genetic mutation.
[24] The system according to [23], wherein the cell is not from a subject or a non-subject.
[25] The system according to [23], wherein the cell is collected from blood.

The invention also relates to the followings:
[1] A method for selecting a neoantigen derived from a subject, comprising: (1) a step of acquiring sequence data of a normal cell and a cancer cell derived from the subject, (2) a step of identifying a gene having a genetic mutation specific to the cancer cell by mutually comparing the sequence data of the normal cell and the cancer cell, and (3) a step of identifying a peptide based on a wild-type gene corresponding to the gene having the genetic mutation specific to the cancer cell from a major histocompatibility complex (MHC)-presented peptide database, wherein the genetic mutation specific to the cancer cell is a missense mutation, and the peptide based on the wild-type gene corresponding to the gene having the genetic mutation specific to the cancer cell has a wild-type amino acid corresponding to the position of an amino acid mutation due to the genetic mutation.
[2] The method according to [1], wherein the sequence data is whole exome sequence data.
[3] The method according to [1] or [2], wherein the MHC-presented peptide database comprises a list of wild-type MHC-presented peptides for each MHC class and/or each organ type.
[4] The method according to [1], wherein the MHC-presented peptide database comprises a list of wild-type MHC-presented peptides.
[5] The method according to any one of [1] to [4], wherein step (3) is a step of identifying a peptide based on a wild-type gene corresponding to the gene having the genetic mutation specific to the cancer cell, from the MHC-presented peptide database for each MHC class and/or each organ type.
[6] The method according to any one of [1] to [5], wherein the amino acid mutation due to the genetic mutation is a single amino acid mutation.
[7] The method according to any one of [1] to [6], wherein the peptide based on the wild-type gene corresponding to the gene having the genetic mutation specific to the cancer cell is scored based on its MHC presentation frequency in the MHC-presented peptide database.
[8] The method according to [7], wherein the peptide based on the wild-type gene corresponding to the gene having the genetic mutation specific to the cancer cell is identified based on the scoring.
[9] The method according to any one of [1] to [8], wherein the MHC-presented peptide database is created by an MHC-presented peptide analysis method comprising a step of obtaining an MHC-peptide complex from a cell, a step of obtaining a peptide from said complex, and a step of identifying the amino acid sequence of said peptide by mass spectrometry.
[10] The method according to any one of [1] to [9], wherein the cell is not from a subject or a non-subject.
[11] The method according to any one of [1] to [9], wherein the cell is collected from blood.
[12] The method according to any one of [1] to [9], wherein the MHC is MHC class I and/or class II.
[13] The method according to any one of [1] to [12], wherein the MHC is a human leukocyte antigen (HLA).
[14] The method according to any one of [1] to [13], further comprising, in the peptide based on the wild-type gene corresponding to the gene having the genetic mutation specific to the cancer cell, said peptide having a wild-type amino acid corresponding to the position of an amino acid mutation due to the genetic mutation, a step of introducing an amino acid mutation due to the genetic mutation and/or a step of determining the gene sequence encoding the peptide into which the amino acid mutation has been introduced.
[15] The method according to [14], further comprising a step of selecting the peptide into which the amino acid mutation has been introduced and/or the nucleic acid consisting of the base sequence into which the genetic mutation has been introduced as a neoantigen derived from the subject.
[16] A method for producing a peptide containing a neoantigen derived from a subject, comprising a step of adding 0 or more optional amino acids to the N-terminus and/or C-terminus of the neoantigen derived from the subject selected by the method according to [1] to [15], wherein the number of amino acids contained in the neoantigen to which the amino acids have been added is 40 or less.
[17] A peptide obtained by the method according to [16].
[18] A method for producing a nucleic acid containing a neoantigen derived from a subject, comprising a step of adding 0 or more optional bases to the 5' end and/or 3' end of a nucleic acid containing the neoantigen derived from the subject selected by the method according to [1] to [15].
[19] A nucleic acid containing a neoantigen derived from a subject, wherein the neoantigen is selected by the method according to [15].
[20] The method according to [18], further comprising a step of producing an mRNA containing a nucleic acid containing a neoantigen derived from the subject.
[21] An mRNA containing a neoantigen derived from a subject, wherein the neoantigen is selected by the method according to [15].
[22] A vaccine composition comprising the peptide according to [17], the nucleic acid according to [19], or the mRNA according to [21].
[23] A system for selecting a neoantigen derived from a subject, comprising: (1) a sequence data acquisition section to acquire sequence data of a normal cell and a cancer cell derived from the subject, (2) a gene identification section to identify a gene having a genetic mutation specific to the cancer cell by mutually comparing the sequence data of the normal cell and the cancer cell, and (3) a peptide identification section to identify a peptide based on a wild-type gene corresponding to the gene having the genetic mutation specific to the cancer cell from a major histocompatibility complex (MHC)-presented peptide database, wherein the genetic mutation specific to the cancer cell is a missense mutation, and the peptide based on the wild-type gene corresponding to the gene having the genetic mutation specific to the cancer cell has a wild-type amino acid corresponding to the position of an amino acid mutation due to the genetic mutation.
[24] The system according to [23], wherein the cell is not from a subject or a non-subject.
[25] The system according to [23] or [24], wherein the cell is collected from blood.

### [Advantageous Effects of Invention]

According to the present invention, neoantigens derived from a subject can be selected. In particular, according to the present invention, by using a combination of conventional in silico method and MS method, the advantages of high sensitivity and simplicity of the former and high specificity of the latter can be simultaneously obtained. That is, according to the present invention, subject-derived neoantigens can be selected with high sensitivity, high specificity, and simplicity. In other words, the present invention allows for efficient, low-cost, and rapid selection of subject-derived neoantigens. In other words, according to the present invention, subject-derived neoantigens can be selected accurately, easily, and rapidly.

For certain cancers, it is extremely difficult to collect tumor biopsies from an organ and to isolate the cells. According to the present invention, it is sufficient to obtain a small number of cells from the cancer and control normal tissues for sequencing analysis that aims at identifying genetic mutations specific to the cancer cells. Cells used for MS analysis, i.e., used for producing a surrogate peptidome, may be cells obtained from tissues other than the subject's cancer tissue, e.g., cells derived from blood, and such cells are generally easier and faster to collect and isolate. That is, the surrogate peptidome can be produced from, but not limited to, the subject's own blood cells, third-party blood cells, or third-party cancer cells. In the present invention, by mutually comparing sequence data of cells collected from subject's cancer biopsy tissue and non-cancerous tissue, genes having genetic mutations specific to cancer cells are identified, and wild-type genes corresponding to the identified genes are collated with the surrogate immunopeptidome to select MHC-presented peptides based on said genes, and the genetic mutations are introduced into said peptides, thereby enabling rapid and easy selection of neoantigens derived from the subject with high sensitivity and specificity. Such a method may also be referred to as NESSIE (Neoantigen Selection using Surrogate Immunopeptidome) in the present specification.

Generally, a relatively large number of cells are required for MS analysis including creation of immunopeptidome, and cells derived from blood can be collected in large numbers. This is a great advantage when it is highly necessary to obtain normal cells from a subject patient, such as when dealing with minor histocompatibility antigens or HLA class II. In addition, even when a sufficient number of cells cannot be collected, a large number of specimens can be obtained from a small number of cells by using EBV viral amplification in blood-derived cells.

The cells to be collected are not limited to those derived from blood, as long as a relatively large number of cells can be collected for MS analysis including immunopeptidome creation.

According to the present invention, if the MHC matches, any of the surrogate immunopeptides can identify both MHC class I and class II neoantigens. For example, a surrogate immunopeptidome derived from patient's own blood cells (immortalized) can be used to identify not only HLA class II, but also both HLA class I and class II neoantigens. Even with surrogate immunopeptides derived from another person's tumor or normal tissue, both HLA class I and class II neoantigens can be identified if the HLA matches.

Furthermore, according to the present invention, based on a neoantigen derived from a selected subject, peptides and/or nucleic acids containing the neoantigen can be produced. In addition, according to the present invention, based on a neoantigen derived from a selected subject, peptides and/or nucleic acids containing the neoantigen can be produced, which can then be applied to personalized medicine for the subject. Furthermore, according to the present invention, based on a neoantigen derived from a selected subject, mRNAs containing the neoantigen can be produced, which can then be applied to personalized medicine for the subject.

### [Brief Description of Drawings]

[Figure 1] Figure 1 shows the concept of the neoantigen selection method of the invention.
[Figure 2] Figure 2 shows the principle of the neoantigen selection method of the invention.
[Figure 3] Figure 3 shows the characteristics of the neoantigen selection method of the invention.
[Figure 4] Figure 4 shows neoantigens derived from mouse colorectal cancer cell line selected by the neoantigen selection method of the invention.
[Figure 5] Figure 5 shows the antitumor effect of administration of neoantigens derived from mouse colorectal cancer cell line selected by the neoantigen selection method of the invention.
[Figure 6] Figure 6 shows the antitumor effect of combined use of neoantigens derived from mouse colorectal cancer cell line selected by the neoantigen selection method of the invention and anti-PD-1 antibodies.
[Figure 7] Figure 7 shows the induction of CD8-positive T cells for neoantigens derived from mouse colorectal cancer cell line selected by the neoantigen selection method of the invention.
[Figure 8] Figure 8 shows neoantigens derived from a panel of human colorectal cancer tissue (CRC) selected by the neoantigen selection method of the invention. Figure 8A shows the number of neoantigens (top row) and the number of missense mutations (bottom row) the patient has in each CRC case of HLA-A24. Figure 8B shows the number of neoantigens (top row) and the number of missense mutations (bottom row) the patient has in each CRC case of HLA-A02.
[Figure 9] Figure 9 shows the tumor-infiltrating lymphocyte (TIL) response to neoantigens derived from a panel of human colorectal cancer tissue (CRC) selected by the neoantigen selection method of the invention. Figure 9A shows the TIL response to the TUBB-RAF9 neoantigens from CRC111 case (HLA-A24). Figure 9B shows the TIL response to the STT3A-KRV9 neoantigens from CRC135 case (HLA-A02).
[Figure 10] Figure 10 shows the reaction of HLA class II-presented neoantigens selected by the neoantigen selection method of the invention with CD4-positive T cells. Figure 10A shows the reaction of SCO1 neoantigens from CRC66 case (HLA-DR*04:06) with patient-derived CD4-positive T cell clones. Figure 10B shows the reaction of said CD4-positive T cell clones with DRB1*04:06-presented neoantigens.
[Figure 11] Figure 11 shows that the combined use of a vaccine against neoantigens selected by the neoantigen selection method of the invention and ICB increases neoantigen-reactive CD8+ cells in the spleen.
[Figure 12] Figure 12 shows an overview of the neoantigen selection method using NESSIE of the invention.
[Figure 13] Figure 13 shows an overview of the selection of HLA class II neoantigens using autologous peripheral blood.
[Figure 14] Figure 14 shows that patient's peripheral blood CD4+ T cells strongly respond to detected neoantigens.

Unless otherwise defined herein, all technical and scientific terms used herein have the same meaning as commonly understood by one of ordinary skill in the art. All patents, applications and other publications and information referenced herein are hereby incorporated by reference in their entirety.

### [Embodiment for Carrying out Invention]

### [Methods for selecting subject-derived neoantigens]

One aspect of the invention is a method for selecting a neoantigen derived from a subject, comprising (1) a step of acquiring sequence data of a normal cell and a cancer cell derived from the subject, (2) a step of identifying a gene having a genetic mutation specific to the cancer cell by mutually comparing the sequence data of the normal cell and the cancer cell, and (3) a step of identifying a peptide based on a wild-type gene corresponding to the gene having the genetic mutation specific to the cancer cell from a major histocompatibility complex (MHC)-presented peptide database, wherein the genetic mutation specific to the cancer cell is a missense mutation, and the peptide based on the wild-type gene corresponding to the gene having the genetic mutation specific to the cancer cell has a wild-type amino acid corresponding to the position of an amino acid mutation due to the genetic mutation (sometimes referred to as the "neoantigen selection method of the invention").

Another aspect of the invention relates to a method for selecting a neoantigen derived from a subject, comprising (1) a step of acquiring sequence data of a cell derived from the subject, (2) a step of identifying a gene having a genetic mutation specific to a cancer cell by mutually comparing the sequence data of said cell and cells derived from the subject or a non-subject by using an MHC-presented peptide database, and (3) a step of identifying a peptide based on a wild-type gene corresponding to the gene having the genetic mutation specific to the cancer cell from a major histocompatibility complex (MHC class)-presented peptide database, wherein the MHC is MHC class I and II, said cell may be derived from blood, the neoantigen-expressing cancer cell may be derived from an organ different from the organ from which said cell was collected, the genetic mutation specific to the cancer cell is a missense mutation, and the peptide based on the wild-type gene corresponding to the gene having the genetic mutation specific to the cancer cell has a wild-type amino acid corresponding to the position of an amino acid mutation due to the genetic mutation (sometimes referred to as NESSIE (Neoantigen Selection using Surrogate Immunopeptidome)).

In the present invention, "subject" refers to any animal having major histocompatibility complex (MHC) in cells in the living body and has cancer cells in the living body. For example, the subject is a vertebrate such as a mammal, bird, or reptile. For example, the subject is a mammal such as a human, mouse, or rat. Preferably, the subject is a human.

In the present invention, "cancer" refers to any tumor that adversely affects the subject by infiltrating surrounding tissues, metastasizing, or the like. For example, cancers include adrenocortical cancer, anal cancer, bile duct cancer, bladder cancer, breast cancer, ovarian cancer, cervical cancer, leukemia (acute myeloid leukemia, chronic myeloid leukemia, acute lymphocytic leukemia, chronic lymphocytic leukemia, etc.), myeloproliferative tumors (chronic myeloid leukemia, chronic neutrophilic leukemia, polycythemia vera, primary myelofibrosis, essential thrombocythemia, chronic eosinophilic leukemia, etc.), colorectal cancer, endometrial cancer, esophageal cancer, Ewing's tumor, gallbladder cancer, Hodgkin's disease, head and neck cancer, oral cavity cancer, liver cancer, small cell lung cancer, non-small cell lung cancer, malignant lymphoma (non-Hodgkin lymphoma, etc.), melanoma, mesothelioma, multiple myeloma, ovarian cancer, pancreatic cancer, prostate cancer, renal cancer, stomach cancer, testicular cancer, brain tumor, neuroendocrine tumor, neuroendocrine carcinoma (small cell cancer, etc.), and others.

In the present invention, "cancer" includes blood cancer. For example, blood cancer includes leukemia (acute myeloid leukemia, chronic myeloid leukemia, acute lymphatic leukemia, chronic lymphatic leukemia, etc.), myeloproliferative tumors (chronic myeloid leukemia, chronic neutrophilic leukemia, polycythemia vera, primary myelofibrosis, essential thrombocythemia, chronic eosinophilic leukemia, etc.), and others.

In the present invention, "tumor" refers to a cell population formed by autonomous overgrowth of cells against biological control of the living body.

In the present invention, "neoantigen" refers to any protein having a mutation that is not present in normal cells but is specifically present only in cancer cells, any peptide derived from said protein (said peptide having a mutation that is specifically present only in the cancer cells), or any nucleic acid consisting of a base sequence encoding said protein or said peptide, and in particular, refers to a peptide derived from said protein or a nucleic acid consisting of a base sequence encoding said peptide.

Genetic mutations that result in neoantigens are only marginally present in the vast number of genetic mutations in cancer cells. In addition, since such genetic mutations vary from subject to subject, neoantigens also vary from subject to subject.

Cancer cells present neoantigens to CD8-positive T cells via MHC class I molecules on the surface of the cancer cells. CD8-positive T cells recognize and activate neoantigens via TCRs, thereby distinguishing between normal cells and cancer cells and specifically eliminating the cancer cells. That is, by inducing CD8-positive T cells to recognize neoantigens via TCRs, the immune response against cancer can be specifically enhanced and highly specific immunotherapy can be realized. In particular, since neoantigens are different in each subject, by inducing CD8-positive T cells to recognize neoantigens via TCRs, the immune response against cancer can be specifically enhanced in each subject, and highly specific immunotherapy personalized for each subject can be realized.

In addition, since neoantigens have mutations that are not present in normal cells but only specifically in cancer cells, and they do not originally exist in the living body, immune tolerance in the thymus can be avoided. Thus, it is expected that inducing CD8-positive T cells to recognize neoantigens via TCRs will induce a strong immune response.

In the present invention, "normal cell" refers to any cells that do not cause cancer in a subject by growing in accordance with in vivo controls.

In the present invention, "cancer cell" refers to any cells that cause cancer in a subject by growing autonomously and excessively against in vivo controls.

Normal cells and cancer cells originate from any tissue and organ of a subject. For example, normal cells and cancer cells originate from the digestive system (esophagus, stomach, small intestine, large intestine, liver, gallbladder, pancreas, etc.), circulatory system (blood, blood vessels, lymph vessels, lymph nodes, spleen, thymus, etc.), respiratory system (trachea, bronchus, lung, etc.), urinary system (kidney, ureter, bladder, urethra, etc.), reproductive system (testis, prostate, ovary, uterus, etc.), endocrine system (pituitary gland, thyroid gland, adrenal gland, pancreas, etc.), nervous system (brain, spinal cord, etc.), or motor system (bone, cartilage, skeletal muscle, etc.). Normal cells may originate from organs other than the organ with cancer in a subject.

In one embodiment of the invention, normal cells and/or cancer cells may be derived from blood.

In the present invention, "sequence data" refers to the base sequence of nucleotides constituting any nucleic acid.

In the present invention, "mutually comparing sequence data" refers to arranging (aligning) two or more types of sequence data so that similar regions of nucleotide base sequences can be identified. For example, nucleotide base sequences are expressed as rows of a matrix, and gaps are inserted so that sequences with identical or similar nature are arranged in the same column.

In the present invention, "cancer cell-specific genetic mutation (or genetic mutation specific to cancer cells)" refers to any genetic mutation that occurs only in cancer cells of a subject and not in normal cells of the subject.

In the present invention, "wild-type gene corresponding to a gene having a cancer cell-specific genetic mutation" refers to the gene before the cancer cell-specific genetic mutation occurs.

In the present invention, "peptide based on a wild-type gene corresponding to a gene having a cancer cell-specific genetic mutation" refers to a peptide encoded by the base sequence in a wild-type gene corresponding to a gene having a cancer cell-specific genetic mutation.

In the present invention, "major histocompatibility complex (MHC)" refers to a transmembrane glycoprotein that resides on the cell surface and presents peptides derived from intracellular and extracellular proteins. MHC is primarily classified as Class I and Class II. Most nucleated cells in the living body have MHC class I molecules, and intracellular protein-derived peptides can be presented to CD8-positive T cells via MHC class I molecules. On the other hand, antigen-presenting cells have MHC class II molecules, and peptides derived from extracellular proteins can be presented to CD4-positive T cells via MHC class II molecules.

In the present invention, "major histocompatibility complex (MHC)-presented peptide database" refers to a database comprising the amino acid sequence of each MHC-presented peptide, created by an MHC-presented peptide analysis method that comprises a step of obtaining an MHC-peptide complex from any cell, a step of obtaining a peptide from said complex, and a step of identifying the amino acid sequence of said peptide by mass spectrometry. The MHC-presented peptide database contains the amino acid sequences of peptides actually presented by MHC in the living body. In addition, such peptides are abundant in the living body. Thus, CD8-positive T cells tend to be induced to recognize such peptides via TCRs in the living body.

In the present invention, "missense mutation" refers to the replacement of an original amino acid (wild-type amino acid) with a different amino acid (mutated amino acid) by the replacement of a base in a codon.

In the present invention, "position of an amino acid mutation due to a cancer cell-specific genetic mutation" refers to, in a gene having a cancer cell-specific genetic mutation, the position of an amino acid encoded by a base sequence that is present at the position where the genetic mutation is occurring.

In the present invention, "wild-type amino acid corresponding to the position of an amino acid mutation due to a cancer cell-specific genetic mutation" refers to, in a gene before a cancer cell-specific genetic mutation occurs, the amino acid encoded by a base sequence that is present at the position where the genetic mutation will occur.

In the present invention, "a peptide based on a wild-type gene corresponding to a gene having a cancer cell-specific genetic mutation has a wild-type amino acid corresponding to the position of an amino acid mutation due to the genetic mutation" means that a peptide encoded by a base sequence in a wild-type gene corresponding to the gene having a cancer cell-specific genetic mutation has, in the gene before the cancer cell-specific genetic mutation occurs, an amino acid encoded by a base sequence that is present at the position where the genetic mutation will occur.

In the present invention, "a peptide based on a gene having a cancer cell-specific genetic mutation (mutant peptide)" and "a peptide based on a wild-type gene corresponding to a gene having a cancer cell-specific genetic mutation (wild-type peptide)" can be represented as follows, respectively.
Mutant peptide: (X)ₘ-Mt-(X)ₙ
Wild-type peptide: (X)ₘ-Wt-(X)ₙ
where Mt is a mutant amino acid, "Wt" is a wild-type amino acid corresponding to the mutant amino acid, X is each independently any wild-type amino acid, and m and n are each independently any integer of 0 or more. In addition, (X)ₘ is located on the N-terminus side and (X)ₙ is located on the C-terminus side. For example, m and n are each independently any integer from 0 to 20, 0 to 15, 0 to 10, or 0 to 5, and the sum of m and n is any integer of 40 or less, 30 or less, 20 or less, or 10 or less.

In one embodiment, the sequence data is whole exome sequence data.

In the present invention, "whole exome sequence data" refers to information on base sequences obtained by selectively obtaining exon regions that code for proteins in the genome of any cell, and determining the sequence of such regions using a next-generation sequencer. Whole exome sequence data can be used to efficiently search for cancer-related genetic mutations.

In one embodiment, the MHC-presented peptide database comprises a list of wild-type MHC-presented peptides for each MHC class and/or each organ type.

In the present invention, "comprising a list of wild-type MHC-presented peptides for each MHC class and/or each organ type" refers to comprising a list of wild-type MHC-presented peptides, i.e., peptides actually presented by MHC in a living body, classified and listed for each MHC class and/or each organ type from which each peptide is derived.

In another embodiment, the MHC-presented peptide database comprises a list of wild-type MHC-presented peptides, for both MHC Class I and MHC Class II.

In one embodiment, step (3) is a step of identifying a peptide based on a wild-type gene corresponding to the gene having the genetic mutation specific to the cancer cell, from an MHC-presented peptide database for each MHC class and/or each organ type.

In the present invention, "identifying from an MHC-presented peptide database for each MHC class and/or each organ type" refers to, after identifying the MHC class and/or the type of organ with cancer of a subject, a peptide based on a wild-type gene corresponding to the gene having the genetic mutation specific to the cancer cell is identified from the MHC-presented peptide database such that the MHC class and/or the organ type of the subject matches an MHC class and/or an organ type in the MHC-presented peptide database.

In order to identify the MHC class of a subject, any cell of the subject may be used. For example, such a cell may be derived from an organ other than the organ with cancer of the subject.

In another embodiment, step (3) is a step of identifying a peptide based on a wild-type gene corresponding to the gene having the genetic mutation specific to the cancer cell, from a database including a database of MHC class I and MHC class II MHC-presented peptides, wherein the cell may be derived from blood.

In one embodiment, the amino acid mutation due to the genetic mutation is a single amino acid mutation.

In one embodiment, the peptides based on the wild-type gene corresponding to the gene having the cancer cell-specific genetic mutation are scored based on the respective MHC presentation frequencies in the MHC-presented peptide database.

In the present invention, "scored based on the respective MHC presentation frequencies in the MHC-presented peptide database" refers to the following: regarding the peptide based on a wild-type gene corresponding to a gene having a cancer cell-specific genetic mutation (i.e., the above-mentioned "wild-type peptide") which has matched "Wt" and amino acid sequences (i.e., the above-mentioned "(X)ₘ" and "(X)ₙ") at its N-terminus side and C-terminus side, whether or not said peptide is registered in the MHC-presented peptide database is examined; and if said peptide is registered in the MHC-presented peptide database, this is regarded as a "hit," and if said peptide is registered once in the MHC-presented peptide database, this is regarded as "1 hit," and for each 1 hit, a maximum of 1 point is added to the score of said peptide. Conversely, if said peptide is not registered in the MHC-presented peptide database, the score for said peptide is 0 points.

In one embodiment, the peptide based on the wild-type gene corresponding to the gene having the genetic mutation specific to the cancer cell is identified based on the above-mentioned scoring.

In the present invention, "identified based on the scoring" refers to, when the score is greater than 0, listing the respective amino acid sequence and calculated score for each peptide that is registered in the MHC-presented peptide database. From the viewpoint of effectiveness of neoantigens in the living body, a score of 1 or more is preferable, 2 or more is more preferable, and 3 or more is even more preferable.

In one embodiment, the MHC-presented peptide database is created by an MHC-presented peptide analysis method comprising a step of obtaining an MHC-peptide complex from a cell, a step of obtaining a peptide from said complex, and a step of identifying the amino acid sequence of said peptide by mass spectrometry.

In the present invention, "MHC-peptide complex" refers to an MHC presenting a peptide derived from an intracellular or extracellular protein.

In one embodiment, the MHC is MHC class I and/or class II.

In one embodiment, the MHC is a human leukocyte antigen (HLA).

In the present invention, "human leukocyte antigen (HLA)" refers to human MHC. HLAs are primarily classified into Class I and Class II. Class I is further classified into HLA-A, HLA-B, HLA-C, etc., and Class II is further classified into HLA-DR, HLA-DQ, HLA-DP, etc.

In one embodiment, the neoantigen selection method of the invention further comprises a step of identifying a base sequence encoding the peptide based on the wild-type gene corresponding to the gene having the genetic mutation specific to the cancer cell.

In one embodiment, the neoantigen selection method of the invention further comprises a step of introducing an amino acid mutation due to the genetic mutation in the peptide based on the wild-type gene corresponding to the gene having the genetic mutation specific to the cancer cell, and/or a step of introducing the genetic mutation in the base sequence encoding said peptide.

In the present invention, "introducing an amino acid mutation due to a genetic mutation specific to cancer cells" refers to replacing "Wt" with "Mt" in a wild-type peptide registered in an MHC-presented peptide database.

In the present invention, "introducing a genetic mutation specific to cancer cells" refers to replacing a base sequence corresponding to "Wt" with a base sequence corresponding to "Mt" in a base sequence encoding a wild-type peptide registered in an MHC-presented peptide database.

In one embodiment, the neoantigen selection method of the invention further comprises a step of selecting a peptide into which the amino acid mutation has been introduced and/or a nucleic acid consisting of the base sequence into which the genetic mutation has been introduced as a neoantigen derived from the subject.

Neoantigens derived from a subject selected by the neoantigen selection method of the invention can be applied to immunotherapy such as cancer vaccine therapy and genetically engineered T cell therapy. In cancer vaccine therapy, neoantigens are administered to the subject to activate the subject's immune response against cancer. In genetically engineered T cell therapy, the T cells collected from the subject's body are genetically engineered to express a TCR gene that recognizes a neoantigen presented on MHC or a gene for a fusion protein of an antigen-binding site of an antibody recognizing the neoantigen presented on MHC and an activation domain of TCR, then cultured and reintroduced back into the subject to activate the subject's immune response against cancer. Either a genetically engineered T cell therapy using cells (TCR-T cells) genetically engineered to express a gene for TCR that recognizes neoantigens presented on MHC (TCR-T cell therapy), or a genetically engineered T cell therapy using cells (CAR-T cells) genetically engineered to express a gene for a fusion protein of an antigen-binding site of an antibody recognizing the neoantigen presented on MHC and an activation domain of TCR (CAR-T cell therapy), can be utilized.

Furthermore, the neoantigens derived from a subject selected by the neoantigen selection method of the invention can also be used in combination with immune checkpoint therapy. In immune checkpoint therapy, immune checkpoint inhibitors are bound to specific molecules that act to suppress immune responses on the surface of cancer cells, and such suppression of immune responses is prevented, thereby activating the subject's immune response against cancer.

When applied to such immunotherapy, the neoantigens derived from a subject selected by the neoantigen selection method of the invention may be in the form of either a peptide, or a nucleic acid consisting of a base sequence encoding the peptide. In addition, amino acids may also be added to the N-terminus and/or C-terminus of the neoantigens derived from a subject selected by the neoantigen selection method of the invention. In addition, nucleic acids may also be added to the 5' end and/or 3' end of the neoantigens derived from a subject selected by the neoantigen selection method of the invention.

For example, in cancer vaccine therapy, the neoantigens derived from a subject selected by the neoantigen selection method of the invention can be used in various forms such as peptide vaccines, mRNA vaccines, DNA vaccines, viral vector vaccines, virus-like particle vaccines, plasmid DNA vaccines, bacterial vector vaccines, and dendritic cell vaccines.

In peptide vaccines, a peptide containing a neoantigen is administered to a subject, thereby activating the subject's immune response against cancer. In mRNA vaccines, an artificial copy of an mRNA containing a base sequence encoding a neoantigen is administered to a subject, and by expressing the neoantigen in the living body, the subject's immune response against cancer is activated. In DNA vaccines, an artificial copy of a DNA containing a base sequence encoding a neoantigen is administered to a subject, and by expressing the neoantigen in the living body, the subject's immune response against cancer is activated. In viral vector vaccines, a virus containing a nucleic acid consisting of a base sequence encoding a neoantigen is administered to a subject, and by expressing the neoantigen in the living body, the subject's immune response against cancer is activated. In virus-like particle vaccines, a virus-like particle (having an external structure similar to a virus but not having genetic information) containing a nucleic acid consisting of a base sequence encoding a neoantigen is administered to a subject, and by expressing the neoantigen in the living body, the subject's immune response against cancer is activated. In plasmid DNA vaccines, a plasmid DNA containing a base sequence encoding a neoantigen is administered to a subject, and by expressing the neoantigen in the living body, the subject's immune response against cancer is activated. In bacterial vector vaccines, bacteria containing nucleic acids consisting of a base sequence encoding a neoantigen are administered to a subject, and by expressing the neoantigen in the living body, the subject's immune response against cancer is activated. In dendritic cell vaccines, a peptide containing a neoantigen is bound to dendritic cells outside the living body, and such dendritic cells are administered to a subject to activate T cells under conditions closer to a physiological environment, thereby activating the subject's immune response against cancer.

The present invention may also be a nucleic acid containing a neoantigen derived from a subject selected by the neoantigen selection method of the invention. Such a nucleic acid may preferably contain tumor-specific frameworks, tumor-related genes, and/or tumor-specific missense mutations. In yet another aspect, the present invention may be an mRNA containing a neoantigen derived from a subject selected by the neoantigen selection method of the invention. Such an mRNA may preferably contain tumor-specific frameworks, tumor-related genes, and/or tumor-specific missense mutations.

Preferably, the neoantigen derived from a subject selected by the neoantigen selection method of the invention is used in the form of an mRNA vaccine. In addition, from the viewpoint of stability in a living body, it is preferable to introduce a modified nucleoside such as pseudouridine 2'-O-methylated nucleoside into the mRNA.

The above-mentioned characteristics of the neoantigens derived from subjects selected by the neoantigen selection method of the invention also apply to those produced by the neoantigen production method of the invention, the neoantigen peptide production method of the invention, the neoantigen nucleic acid production method of the invention, the neoantigen-containing peptide production method of the invention, and the neoantigen-containing nucleic acid production method of the invention described below.

### [Methods for producing neoantigens derived from subject]

Another aspect of the invention relates to a method for producing a neoantigen derived from a subject, comprising (1) a step of acquiring sequence data of a normal cell and a cancer cell derived from the subject, (2) a step of identifying a gene having a genetic mutation specific to the cancer cell by mutually comparing the sequence data of the normal cell and the cancer cell, (3) a step of identifying a peptide based on a wild-type gene corresponding to the gene having the genetic mutation specific to the cancer cell from a major histocompatibility complex (MHC)-presented peptide database, (4) a step of identifying a base sequence encoding the peptide based on the wild-type gene corresponding to the gene having the genetic mutation specific to the cancer cell, and (5) a step of introducing an amino acid mutation due to the genetic mutation in the peptide based on the wild-type gene corresponding to the gene having the genetic mutation specific to the cancer cell, and/or a step of introducing the genetic mutation in the base sequence encoding the peptide, wherein the genetic mutation specific to the cancer cell is a missense mutation, and the peptide based on the wild-type gene corresponding to the gene having the genetic mutation specific to the cancer cell has a wild-type amino acid corresponding to the position of the amino acid mutation due to the genetic mutation (sometimes referred to as the "neoantigen production method of the invention").

Yet another aspect of the invention relates to a method for producing a neoantigen derived from a subject, comprising (1) a step of acquiring sequence data of a cell derived from the subject, (2) a step of identifying a gene having a genetic mutation specific to a cancer cell by mutually comparing the sequence data of said cell and a cell derived from the subject or non-subject, (3) a step of identifying a peptide based on a wild-type gene corresponding to the gene having the genetic mutation specific to the cancer cell from a database including a major histocompatibility complex-presented peptide database, (4) a step of identifying a base sequence encoding the peptide based on the wild-type gene corresponding to the gene having the genetic mutation specific to the cancer cell, and (5) a step of introducing an amino acid mutation due to the genetic mutation in the peptide based on the wild-type gene corresponding to the gene having the genetic mutation specific to the cancer cell, and/or a step of introducing the genetic mutation in the base sequence encoding the peptide, wherein said cell may be derived from blood, the neoantigen-expressing cancer cell may be derived from an organ different from the organ from which said cell was derived, the MHC may be MHC class I and II, the genetic mutation specific to the cancer cell is a missense mutation, and the peptide based on the wild-type gene corresponding to the gene having the genetic mutation specific to the cancer cell has a wild-type amino acid corresponding to the position of the amino acid mutation due to the genetic mutation (sometimes referred to as the method of neoantigen production by NESSIE of the invention).

In one embodiment, the neoantigen production method of the invention further comprises a step of selecting a peptide into which the amino acid mutation has been introduced and/or a nucleic acid consisting of the base sequence into which the genetic mutation has been introduced as a neoantigen derived from the subject.

In one embodiment, the neoantigen production method of the invention comprises a step of adding 0 or more optional amino acids to the N-terminus and/or C-terminus of the subject-derived neoantigen, wherein the number of amino acids in the neoantigen to which the amino acids have been added is 40 or less.

In the present invention, "adding 0 or more optional amino acids to the N-terminus and/or C-terminus" refers to, for example, adding 0 to 40, 0 to 25, 0 to 20, 0 to 15, 0 to 10, or 0 to 5 optional amino acids to the N-terminus and/or C-terminus. The optional amino acids may be an amino acid based on a wild-type gene corresponding to a gene having a cancer cell-specific genetic mutation, or they may be any artificially selected amino acids.

In the present invention, "the number of amino acids in the neoantigen to which amino acids have been added is 40 or less" means, for example, that the number of amino acids in the neoantigen to which amino acids have been added is 40 or less, 35 or less, 30 or less, 25 or less, 20 or less, or 15 or less.

For neoantigens presented by MHC class I, one or more optional amino acids may be added only to the N-terminus or C-terminus of the neoantigen derived from the subject.

For neoantigens presented by MHC class II, one or more optional amino acids may be added only to the N-terminus or C-terminus of the neoantigen derived from the subject.

In one embodiment, the neoantigen production method of the invention comprises a step of adding 0 or more optional bases to the 5' end and/or 3' end of the neoantigen derived from the subject. The number of bases in the neoantigen to which the bases have been added may be 900 or less.

In the present invention, "adding 0 or more optional bases to the 5' end and/or 3' end" means, for example, 0 to 900, 0 to 750, 0 to 600, 0 to 450, 0 to 30, or 0 to 150 optional bases are added to the 5' end and/or 3' end. The optional bases may be those based on a wild-type gene corresponding to a gene having a cancer cell-specific genetic mutation, or they may be any artificially selected bases.

In the present invention, "the number of bases in the neoantigen to which the bases have been added is 900 or less" means, for example, that the number of bases in the neoantigen to which the bases have been added is 900 or less, 300 or less, 150 or less, 75 or less, 60 or less, or 45 or less.

For neoantigens presented by MHC class I, 3 or more optional bases may be added only to the 5' end and/or 3' end of the neoantigen derived from the subject.

For neoantigens presented by MHC class II, 3 or more optional bases may be added only to the 5' end and/or 3' end of the neoantigen derived from the subject.

From the viewpoint of effectiveness of neoantigens in the living body, it is preferable that in a neoantigen to which amino acids have been added, there are 10 to 14 amino acids on each of the N-terminus side and C-terminus side of "Mt", and that the number of amino acids contained in the neoantigen to which amino acids have been added is 23 to 27.

From the viewpoint of effectiveness of neoantigens in the living body, it is preferable that in a neoantigen to which bases have been added, there are 30 to 42 bases on each of the 5' end and/or 3' end of "Mt", and that the number of bases contained in the neoantigen to which bases have been added is 69 to 81.

### [Methods for producing neoantigen peptides derived from subject]

Another aspect of the invention relates to a method for producing a neoantigen derived from a subject, comprising (1) a step of acquiring sequence data of a normal cell and a cancer cell derived from the subject, (2) a step of identifying a gene having a genetic mutation specific to the cancer cell by mutually comparing the sequence data of the normal cell and the cancer cell, (3) a step of identifying a peptide based on a wild-type gene corresponding to the gene having the genetic mutation specific to the cancer cell from a major histocompatibility complex (MHC)-presented peptide database, and (4) a step of introducing an amino acid mutation due to the genetic mutation in the peptide based on the wild-type gene corresponding to the gene having the genetic mutation specific to the cancer cell, wherein the genetic mutation specific to the cancer cell is a missense mutation, and the peptide based on the wild-type gene corresponding to the gene having the genetic mutation specific to the cancer cell has a wild-type amino acid corresponding to the position of the amino acid mutation due to the genetic mutation (sometimes referred to as the "neoantigen peptide production method of the invention").

Yet another aspect of the invention relates to a method for producing a neoantigen derived from a subject, comprising (1) a step of acquiring sequence data of a cell derived from the subject, (2) a step of identifying a gene having a genetic mutation specific to a cancer cell by mutually comparing the sequence data of said cell and a cell derived from the subject or non-subject, (3) a step of identifying a peptide based on a wild-type gene corresponding to the gene having the genetic mutation specific to the cancer cell from an MHC-presented peptide database, and (4) a step of introducing an amino acid mutation due to the genetic mutation in the peptide based on the wild-type gene corresponding to the gene having the genetic mutation specific to the cancer cell, wherein said cell may be derived from blood, the neoantigen-expressing cancer cell may be derived from an organ different from the organ from which said cell was derived, the MHC may be MHC class I and II, the genetic mutation specific to the cancer cell is a missense mutation, and the peptide based on the wild-type gene corresponding to the gene having the genetic mutation specific to the cancer cell has a wild-type amino acid corresponding to the position of the amino acid mutation due to the genetic mutation (sometimes referred to as the "method of neoantigen peptide production by NESSIE of the invention").

In one embodiment, the neoantigen peptide production method of the invention further comprises a step of selecting a peptide into which the amino acid mutation has been introduced as a neoantigen derived from the subject.

In one embodiment, the neoantigen peptide production method of the invention comprises a step of adding 0 or more optional amino acids to the N-terminus and/or C-terminus of the neoantigen produced by the neoantigen peptide production method of the invention, wherein the number of amino acids in the neoantigen to which the amino acids have been added is 40 or less.

### [Methods for producing neoantigen nucleic acids derived from subject]

Another aspect of the invention relates to a method for producing a neoantigen derived from a subject, comprising (1) a step of acquiring sequence data of a normal cell and a cancer cell derived from the subject, (2) a step of identifying a gene having a genetic mutation specific to the cancer cell by mutually comparing the sequence data of the normal cell and the cancer cell, (3) a step of identifying a peptide based on a wild-type gene corresponding to the gene having the genetic mutation specific to the cancer cell from a major histocompatibility complex (MHC)-presented peptide database, (4) a step of identifying a base sequence encoding the peptide based on the wild-type gene corresponding to the gene having the genetic mutation specific to the cancer cell, and (5) a step of introducing the genetic mutation into the base sequence encoding the peptide based on the wild-type gene corresponding to the gene having the genetic mutation specific to the cancer cell, wherein the genetic mutation specific to the cancer cell is a missense mutation, and the peptide based on the wild-type gene corresponding to the gene having the genetic mutation specific to the cancer cell has a wild-type amino acid corresponding to the position of an amino acid mutation due to the genetic mutation (sometimes referred to as the "neoantigen nucleic acid production method of the invention").

Yet another aspect of the invention relates to a method for producing a neoantigen derived from a subject, comprising (1) a step of acquiring sequence data of a cell derived from the subject, (2) a step of identifying a gene having a genetic mutation specific to a cancer cell by mutually comparing the sequence data of said cell and a cell derived from the subject or non-subject, (3) a step of identifying a peptide based on a wild-type gene corresponding to the gene having the genetic mutation specific to the cancer cell from a major histocompatibility complex (MHC)-presented peptide database, (4) a step of identifying a base sequence encoding the peptide based on the wild-type gene corresponding to the gene having the genetic mutation specific to the cancer cell, and (5) a step of introducing the genetic mutation into the base sequence encoding the peptide based on the wild-type gene corresponding to the gene having the genetic mutation specific to the cancer cell, wherein said cell may be derived from blood, the neoantigen-expressing cancer cell may be derived from a different organ, the MHC may be MHC class I and II, the genetic mutation specific to the cancer cell is a missense mutation, and the peptide based on the wild-type gene corresponding to the gene having the genetic mutation specific to the cancer cell has a wild-type amino acid corresponding to the position of an amino acid mutation due to the genetic mutation (sometimes referred to as the method of neoantigen nucleic acid production by NESSIE of the invention).

In one embodiment, the neoantigen nucleic acid production method of the invention further comprises a step of selecting a nucleic acid consisting of the base sequence into which the genetic mutation has been introduced as a neoantigen derived from the subject.

In one embodiment, the neoantigen nucleic acid production method of the invention comprises a step of adding 0 or more optional bases to the 5' end and/or 3' end of the subject-derived neoantigen produced by the neoantigen nucleic acid production method of the invention. The number of bases in the neoantigen to which the bases have been added may be 900 or less.

### [Methods for producing peptides containing neoantigens derived from subject]

Another aspect of the invention relates to a method for producing a peptide containing a neoantigen derived from a subject, comprising a step of adding 0 or more optional amino acids to the N-terminus and/or C-terminus of a neoantigen derived from a subject selected by the neoantigen selection method of the invention, wherein the number of amino acids contained in the neoantigen to which the amino acids have been added is 40 or less (sometimes referred to as the "neoantigen-containing peptide production method of the invention").

In the present invention, "peptide containing a neoantigen derived from a subject" refers to a neoantigen derived from a subject to which 0 or more optional amino acids have been added to the N-terminus and/or C-terminus, and the number of amino acids in the neoantigen to which the amino acids have been added is 40 or less.

### [Methods for producing nucleic acids containing neoantigens derived from subject]

Another aspect of the invention relates to a method for producing a nucleic acid containing a neoantigen derived from a subject, comprising a step of adding 0 or more optional bases to the 5' end and/or 3' end of a neoantigen derived from a subject selected by the neoantigen selection method of the invention, wherein the number of bases contained in the base-added neoantigen may be 900 or less (sometimes referred to as the "neoantigen-containing nucleic acid production method of the invention").

In the present invention, "nucleic acid containing a neoantigen derived from a subject" refers to a neoantigen derived from the subject to which 0 or more optional bases are added to the 5' end and/or 3' end.

In one embodiment, the neoantigen-containing nucleic acid production method of the invention further comprises a step of producing an mRNA containing neoantigen-containing nucleic acids derived from the subject.

### [System for selecting subject-derived neoantigens]

Another aspect of the invention relates to a system for selecting a neoantigen derived from a subject, comprising: (1) a sequence data acquisition section to acquire sequence data of a normal cell and a cancer cell derived from the subject, (2) a gene identification section to identify a gene having a genetic mutation specific to the cancer cell by mutually comparing the sequence data of the normal cell and the cancer cell, and (3) a peptide identification section to identify a peptide based on a wild-type gene corresponding to the gene having the genetic mutation specific to the cancer cell from a major histocompatibility complex (MHC)-presented peptide database, wherein the genetic mutation specific to the cancer cell is a missense mutation, and the peptide based on the wild-type gene corresponding to the gene having the genetic mutation specific to the cancer cell has a wild-type amino acid corresponding to the position of an amino acid mutation due to the genetic mutation (sometimes referred to as the "neoantigen selection method of the invention").

Yet another aspect of the invention relates to a system for selecting a neoantigen derived from a subject, comprising (1) a sequence data acquisition section to acquire sequence data of a cell derived from the subject, (2) a gene identification section to identify a gene having a genetic mutation specific to a cancer cell by mutually comparing the sequence data of said cell; and (3) a peptide identification section to identify a peptide based on a wild-type gene corresponding to the gene having the genetic mutation specific to the cancer cell from a major histocompatibility complex (MHC)-presented peptide database, wherein said cell may be derived from blood, the neoantigen-expressing cancer cell may be derived from a different organ, the MHC may be MHC class I and II, the genetic mutation specific to the cancer cell is a missense mutation, and the peptide based on the wild-type gene corresponding to the gene having the genetic mutation specific to the cancer cell has a wild-type amino acid corresponding to the position of an amino acid mutation due to the genetic mutation (sometimes referred to as the "method of neoantigen selection by NESSIE of the invention").

In the present invention, "sequence data acquisition section" refers to a part that acquires sequence data of normal cells and cancer cells derived from a subject. The sequence data acquisition section may comprise a sequencer. For example, the sequencer is a next-generation sequencer.

In the present invention, "gene identification section" refers to a part that identifies genes having genetic mutations specific to the cancer cells by mutually comparing sequence data of normal cells and cancer cells. The gene identification section may comprise software that arranges (aligns) two or more types of sequence data so that similar regions of the base sequences of nucleotides can be identified.

In the present invention, "peptide identification section" refers to a part that identifies peptides based on wild-type genes corresponding to genes having genetic mutations specific to cancer cells from an MHC-presented peptide database. The peptide identification section may comprise software that arranges (aligns) amino acid sequences of two or more peptides so that similar regions of the amino acid sequences of the peptides can be identified.

### [System for producing neoantigens derived from subject]

Another aspect of the invention relates to a system for producing a neoantigen derived from a subject, comprising (1) a sequence data acquisition section to acquire sequence data of a normal cell and a cancer cell derived from the subject, (2) a gene identification section to identify a gene having a genetic mutation specific to the cancer cell by mutually comparing the sequence data of the normal cell and the cancer cell, (3) a peptide identification section to identify a peptide based on a wild-type gene corresponding to the gene having the genetic mutation specific to the cancer cell from a major histocompatibility complex (MHC)-presented peptide database, (4) a base sequence identification section to identify a base sequence encoding the peptide based on the wild-type gene corresponding to the gene having the genetic mutation specific to the cancer cell, and (5) an amino acid mutation-introduction section to introduce an amino acid mutation due to the genetic mutation in the peptide based on the wild-type gene corresponding to the gene having the genetic mutation specific to the cancer cell, and/or a genetic mutation introduction section to introduce the genetic mutation in the base sequence encoding the peptide, wherein the genetic mutation specific to the cancer cell is a missense mutation, and the peptide based on the wild-type gene corresponding to the gene having the genetic mutation specific to the cancer cell has a wild-type amino acid corresponding to the position of the amino acid mutation due to the genetic mutation (sometimes referred to as the "neoantigen production system of the invention").

Yet another aspect of the invention relates to a system for producing a neoantigen derived from a subject, comprising (1) a sequence data acquisition section to acquire sequence data of a cell derived from the subject, (2) a gene identification section to identify a gene having a genetic mutation specific to a cancer cell by mutually comparing the sequence data of said cell and a cell derived from the subject or non-subject, (3) a peptide identification section to identify a peptide based on a wild-type gene corresponding to the gene having the genetic mutation specific to the cancer cell from a major histocompatibility complex (MHC)-presented peptide database, (4) a base sequence identification section to identify a base sequence encoding the peptide based on the wild-type gene corresponding to the gene having the genetic mutation specific to the cancer cell, and (5) an amino acid mutation introduction section to introduce an amino acid mutation due to the genetic mutation in the peptide based on the wild-type gene corresponding to the gene having the genetic mutation specific to the cancer cell, and/or a genetic mutation introduction section to introduce the genetic mutation in the base sequence encoding the peptide, wherein said cell may be derived from blood, the neoantigen-expressing cancer cell may be derived from a different organ, the MHC may be MHC class I and II, the genetic mutation specific to the cancer cell is a missense mutation, and the peptide based on the wild-type gene corresponding to the gene having the genetic mutation specific to the cancer cell has a wild-type amino acid corresponding to the position of the amino acid mutation due to the genetic mutation (sometimes referred to as the "system of neoantigen production by NESSIE of the invention").

In the present invention, "base sequence identification section" refers to a part that identifies a base sequence encoding a peptide based on a wild-type gene corresponding to a gene having a cancer cell-specific genetic mutation. The base sequence identification section may comprise a sequencer.

In the present invention, "amino acid mutation introduction section" refers to, in a peptide based on a wild-type gene corresponding to a gene having a cancer cell-specific genetic mutation, a part that introduces an amino acid mutation due to the genetic mutation. The amino acid mutation introduction section may comprise software that replaces "Wt" with "Mt" in a wild-type peptide registered in an MHC-presented peptide database.

In the present invention, "genetic mutation introduction section" refers to, in a base sequence encoding a peptide based on a wild-type gene corresponding to a gene having a genetic mutation specific to a cancer cell, a part that introduces the genetic mutation. The genetic mutation introduction section may comprise software that replaces a base sequence corresponding to "Wt" with a base sequence corresponding to "Mt" in a base sequence encoding a wild-type peptide registered in an MHC-presented peptide database.

In one embodiment, the neoantigen production system of the invention further comprises a neoantigen selection system to select a peptide into which the amino acid mutation has been introduced and/or a nucleic acid consisting of the base sequence into which the genetic mutation has been introduced, as a neoantigen derived from the subject.

In the present invention, the "neoantigen selection section" refers to a part that selects a peptide into which the amino acid mutation has been introduced and/or a nucleic acid consisting of the base sequence into which the genetic mutation has been introduced, as a neoantigen derived from the subject. The neoantigen selection section may comprise software that selects a peptide into which the amino acid mutation has been introduced and/or a nucleic acid consisting of the base sequence into which the genetic mutation has been introduced, as a neoantigen derived from the subject.

In one embodiment, the neoantigen peptide production system of the invention comprises an amino acid addition section that adds 0 or more optional amino acids to the N-terminus and/or C-terminus of a subject-derived neoantigen produced by the neoantigen peptide production system of the invention, wherein the number of amino acids in the neoantigen to which the amino acids have been added is 40 or less.

In the present invention, "amino acid addition section" refers to a part that adds 0 or more optional amino acids to the N-terminus and/or C-terminus of a subject-derived neoantigen produced by the neoantigen production system of the invention. The amino acid addition section may comprise a peptide synthesizer.

In one embodiment, the neoantigen production system of the invention comprises a base addition section that adds 0 or more optional bases to the 5' end and/or 3' end of a subject-derived neoantigen produced by the neoantigen production system of the invention.

In the present invention, "base addition section" refers to a part that adds 0 or more optional bases to the 5' end and/or 3' end of a subject-derived neoantigen produced by the neoantigen production system of the invention. The base addition section may comprise a nucleic acid synthesizer.

### [System for producing neoantigen peptides derived from subject]

Another aspect of the invention relates to a system for producing a neoantigen derived from a subject, comprising (1) a sequence data acquisition section to acquire sequence data of a normal cell and a cancer cell derived from the subject, (2) a gene identification section to identify a gene having a genetic mutation specific to the cancer cell by mutually comparing the sequence data of the normal cell and the cancer cell, (3) a peptide identification section to identify a peptide based on a wild-type gene corresponding to the gene having the genetic mutation specific to the cancer cell from a major histocompatibility complex (MHC)-presented peptide database, and (4) an amino acid mutation introduction section to introduce an amino acid mutation due to the genetic mutation in the peptide based on the wild-type gene corresponding to the gene having the genetic mutation specific to the cancer cell, wherein the genetic mutation specific to the cancer cell is a missense mutation, and the peptide based on the wild-type gene corresponding to the gene having the genetic mutation specific to the cancer cell has a wild-type amino acid corresponding to the position of the amino acid mutation due to the genetic mutation (sometimes referred to as the "neoantigen peptide production system of the invention").

Yet another aspect of the invention relates to a system for producing a neoantigen derived from a subject, comprising (1) a sequence data acquisition section to acquire sequence data of a cell derived from the subject, (2) a gene identification section to identify a gene having a genetic mutation specific to a cancer cell by mutually comparing the sequence data of said cell and a cell derived from the subject or non-subject, (3) a peptide identification section to identify a peptide based on a wild-type gene corresponding to the gene having the genetic mutation specific to the cancer cell from a major histocompatibility complex (MHC)-presented peptide database, and (4) an amino acid mutation introduction section to introduce an amino acid mutation due to the genetic mutation in the peptide based on the wild-type gene corresponding to the gene having the genetic mutation specific to the cancer cell, wherein said cell may be derived from blood, the neoantigen-expressing cancer cell may be derived from a different organ, the MHC may be MHC class I and II, the genetic mutation specific to the cancer cell is a missense mutation, and the peptide based on the wild-type gene corresponding to the gene having the genetic mutation specific to the cancer cell has a wild-type amino acid corresponding to the position of the amino acid mutation due to the genetic mutation (sometimes referred to as the "system of neoantigen peptide production by NESSIE of the invention").

In one embodiment, the neoantigen peptide production system of the invention further comprises a neoantigen selection section that selects the peptide into which the amino acid mutation has been introduced as a neoantigen derived from the subject.

In one embodiment, the neoantigen peptide production system of the invention comprises an amino acid addition section that adds 0 or more optional amino acids to the N-terminus and/or C-terminus of the neoantigen produced by the neoantigen peptide production system of the invention, wherein the number of amino acids in the neoantigen to which the amino acids have been added is 40 or less.

### [System for producing neoantigen nucleic acids derived from subject]

Another aspect of the invention relates to a method for producing a neoantigen derived from a subject, comprising (1) a sequence data acquisition section to acquire sequence data of a normal cell and a cancer cell derived from the subject, (2) a gene identification section to identify a gene having a genetic mutation specific to the cancer cell by mutually comparing the sequence data of the normal cell and the cancer cell, (3) a peptide identification section to identify a peptide based on a wild-type gene corresponding to the gene having the genetic mutation specific to the cancer cell from a major histocompatibility complex (MHC)-presented peptide database, (4) a base sequence identification section to identify a base sequence encoding the peptide based on the wild-type gene corresponding to the gene having the genetic mutation specific to the cancer cell, and (5) a genetic mutation introduction section to introduce the genetic mutation in the base sequence encoding the peptide based on the wild-type gene corresponding to the gene having the genetic mutation specific to the cancer cell, wherein the genetic mutation specific to the cancer cell is a missense mutation, and the peptide based on the wild-type gene corresponding to the gene having the genetic mutation specific to the cancer cell has a wild-type amino acid corresponding to the position of an amino acid mutation due to the genetic mutation (sometimes referred to as the "neoantigen nucleic acid production system of the invention").

Another aspect of the invention relates to a method for producing a neoantigen derived from a subject, comprising (1) a sequence data acquisition section to acquire sequence data of a cell derived from the subject, (2) a gene identification section to identify a gene having a genetic mutation specific to a cancer cell by mutually comparing the sequence data of said cell and a cell derived from the subject or non-subject, (3) a peptide identification section to identify a peptide based on a wild-type gene corresponding to the gene having the genetic mutation specific to the cancer cell from a major histocompatibility complex (MHC)-presented peptide database, (4) a base sequence identification section to identify a base sequence encoding the peptide based on the wild-type gene corresponding to the gene having the genetic mutation specific to the cancer cell, and (5) a genetic mutation introduction section to introduce the genetic mutation in the base sequence encoding the peptide based on the wild-type gene corresponding to the gene having the genetic mutation specific to the cancer cell, wherein said cell may be derived from blood, the neoantigen-expressing cancer cell may be derived from a different organ, the MHC may be MHC class I and II, the genetic mutation specific to the cancer cell is a missense mutation, and the peptide based on the wild-type gene corresponding to the gene having the genetic mutation specific to the cancer cell has a wild-type amino acid corresponding to the position of an amino acid mutation due to the genetic mutation (sometimes referred to as the "system of neoantigen nucleic acid production by NESSIE of the invention").

In one embodiment, the neoantigen nucleic acid production system of the invention further comprises a neoantigen selection section that selects a nucleic acid consisting of the base sequence into which the genetic mutation has been introduced, as a neoantigen derived from the subject.

In one embodiment, the neoantigen nucleic acid production system of the invention comprises a base addition section that adds 0 or more optional bases to the 5' end and/or 3' end of the subject-derived neoantigen produced by the neoantigen nucleic acid production system of the invention. The number of bases in the neoantigen to which the bases have been added may be 900 or less.

### [System for producing peptides containing neoantigens derived from subject]

Another aspect of the invention relates to a system for producing a peptide containing a neoantigen derived from a subject, comprising an amino acid addition section to add 0 or more optional amino acids to the N-terminus and/or C-terminus of the neoantigen derived from the subject selected by the neoantigen selection system of the invention, wherein the number of amino acids in the neoantigen to which the amino acids have been added is 40 or less (sometimes referred to as the "neoantigen-containing peptide production system of the invention").

### [System for producing nucleic acids containing neoantigens derived from subject]

Another aspect of the invention relates to a system for producing a nucleic acid containing a neoantigen derived from a subject, comprising a base addition section to add 0 or more optional bases to the 5' end and/or 3' end of the neoantigen derived from the subject selected by the neoantigen selection system of the invention, wherein the number of bases contained in the base-added neoantigen may be 900 or less (sometimes referred to as the "neoantigen-containing nucleic acid production system of the invention").

In one embodiment, the neoantigen-containing nucleic acid production system of the invention further comprises an mRNA production section to produce an mRNA containing nucleic acids containing neoantigens derived from the subject.

In the present invention, "mRNA production section" refers to the section that produces mRNA containing nucleic acids containing neoantigens derived from the subject. The mRNA production section may comprise an RNA synthesizer.

The present invention will now be described in more detail with reference to the following examples, which illustrate particular embodiments of the invention and are not intended to be limiting the invention.

### [Examples]

### [Concept of the neoantigen selection method of the invention]

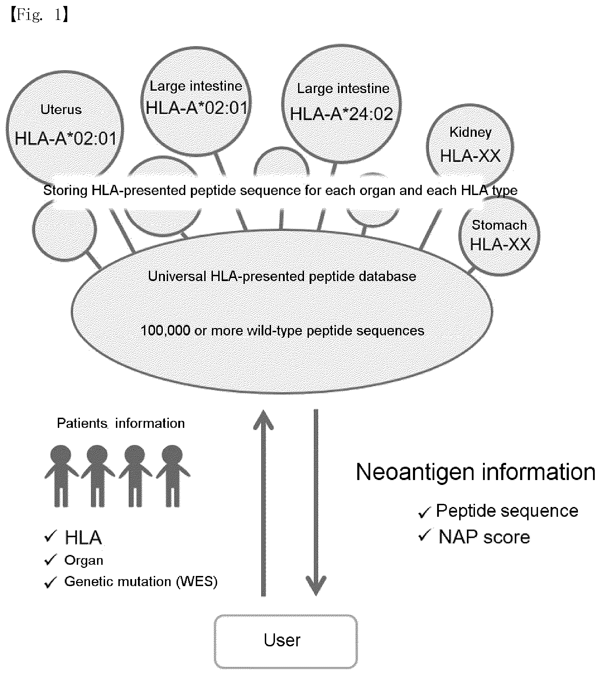

The concept of the neoantigen selection method of the invention is shown in Figure 1. Various embodiments shown in Figure 1 are particular specific examples of the invention and the invention is not limited thereto. "HLA" can also be used interchangeably with "MHC".

First, based on information on a subject such as a patient (HLA class, type of organ with cancer, and cancer cell-specific genetic mutation), a user such as a medical professional identifies peptides based on wild-type genes corresponding to genes having cancer cell-specific genetic mutations from an HLA-presented peptide database for each HLA class and each organ type. Here, the cancer cell-specific genetic mutation is a missense mutation, and the peptide based on the wild-type gene corresponding to the gene having the cancer cell-specific genetic mutation has a wild-type amino acid corresponding to the position of an amino acid mutation due to the genetic mutation.

The gene having the cancer cell-specific genetic mutation is identified by mutually comparing whole exome sequencing (WES) data of normal cells and cancer cells. The HLA-presented peptide database is created by an HLA-presented peptide analysis method comprising a step of obtaining an HLA-peptide complex from a cell, a step of obtaining a peptide from the complex, and a step of identifying the amino acid sequence of the peptide by mass spectrometry, and it comprises a list of wild-type HLA-presented peptides for each HLA class and each organ type.

Next, the peptides based on the wild-type gene corresponding to the gene having the cancer cell-specific genetic mutation are scored based on their respective HLA presentation frequencies in the HLA-presented peptide database, and identified based on the scoring. A peptide produced by introducing an amino acid mutation due to the genetic mutation into the peptides based on the wild-type gene corresponding to the gene having the cancer cell-specific genetic mutation is selected as a neoantigen derived from the subject.

More specifically, said HLA-presented peptide database can be created by a proteogenomics HLA ligandome analysis technology that combines HLA immunoprecipitation, mass spectrometry, and whole exome transcriptome analysis. Such analytical techniques can reveal the overall picture of the HLA ligandome from a sample derived from human tissue.

The present inventors have so far identified over 100,000 natural HLA-presented peptides (i.e., peptides actually presented to HLA in the living body) from clinical samples using proteogenomics HLA ligandome analysis technology. In parallel with this, we generated a panel of cancer antigens to examine tumor-infiltrating lymphocyte (TIL) responses in patients, and obtained an interesting finding that TIL responses were more common for peptides identified by proteogenomics HLA ligandome analysis technology than for peptides predicted by conventional in silico methods. Since peptides identified by proteogenomics HLA ligandome analysis technology are natural HLA-presented peptides and are abundant in the living body, CD8-positive T cells tend to be induced to recognize the peptides via TCRs in the living body.

The same tendency is also seen for neoantigens. In other words, since neoantigens identified by proteogenomics HLA ligandome analysis technology are natural HLA-presented peptides and are abundant in the living body, CD8-positive T cells tend to be induced to recognize the neoantigens in the living body via TCRs.

In addition, since neoantigens have mutations that are not present in normal cells but only specifically in cancer cells, and they do not originally exist in the living body, immune tolerance in the thymus can be avoided. Thus, it is expected that inducing CD8-positive T cells to recognize neoantigens via TCRs will induce a strong immune response.

In other words, neoantigens identified by proteogenomics HLA ligandome analysis technology are thought to have high immunogenicity by efficiently inducing TIL responses in patients. It is also believed that such neoantigens can induce TIL-nonresponsive tumors (cold tumors) to become TIL-responsive tumors (hot tumors) by efficiently inducing TIL response in patients.

In order to effectively utilize the above phenomena, we created an HLA-presented peptide database from data obtained by proteogenomics HLA ligandome analysis technology. Then, based on such database, we constructed a neoantigen selection method of the invention. Since this neoantigen selection method of the invention utilizes an accumulated existing HLA-presented peptide database, it can select neoantigens derived from a subject with high sensitivity, high specificity, and simplicity based only on WES data of normal cells and cancer cells.

### [Principle of the neoantigen selection method of the invention]

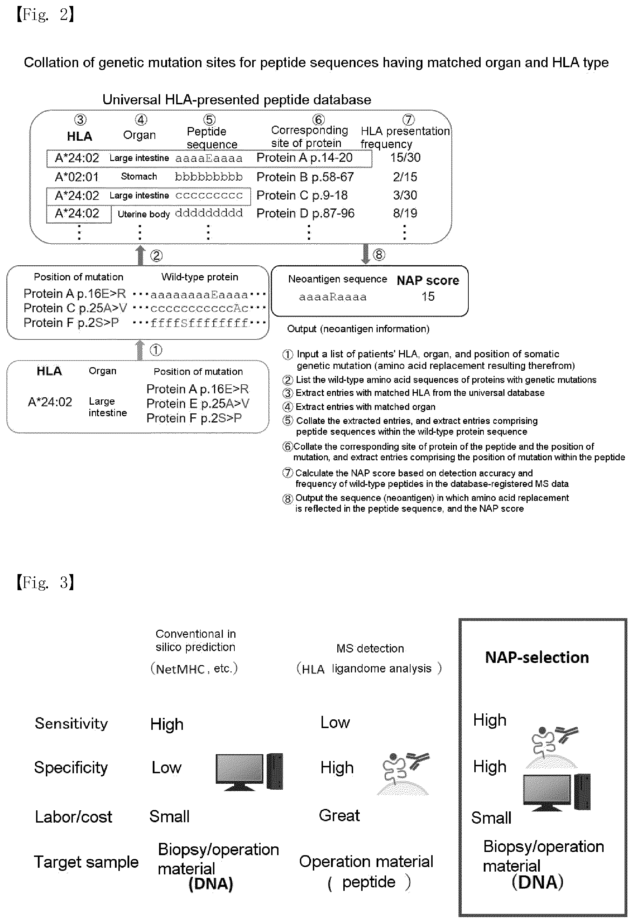

The principle of the neoantigen selection method of the invention is shown in Figure 2. Various embodiments shown in Figure 2 are particular specific examples of the invention and the invention is not limited thereto. In addition, "HLA" may be used interchangeably with "MHC".

The principle of the neoantigen selection method of the invention is to select a neoantigen by collating peptides based on wild-type genes corresponding to genes having cancer cell-specific genetic mutations of a subject such as a patient with natural HLA-presented peptides in an accumulated existing HLA-presented peptide database. At this time, peptides based on wild-type genes corresponding to genes having cancer cell-specific genetic mutations are identified from the HLA-presented peptide database for each HLA class and each organ type. This is based on the fact that when the HLA class and the organ type match between different individuals, the same peptides are presented to HLA with a high frequency.

First, genes having genetic mutations specific to cancer cells derived from a subject such as a patient are identified by mutually comparing the WES data of normal cells and cancer cells. Next, only genes having missense mutations are extracted from the genes having genetic mutations specific to the cancer cells. Next, amino acid sequences based on the base sequences of the genes having such missense mutations are listed, and an mutant amino acid in the amino acid sequences is designated as "Mt", and a wild-type amino acid corresponding to the mutant amino acid is designated as "Wt". That is, a peptide based on the gene having the genetic mutation specific to the cancer cell (mutant peptide) and a peptide based on the wild-type gene corresponding to the gene having the genetic mutation specific to the cancer cell (wild-type peptide) are represented as follows, respectively.
Mutant peptide: (X)ₘ-Mt-(X)ₙ
Wild-type peptide: (X)ₘ-Wt-(X)ₙ

Here, X is independently any wild-type amino acid, and m and n are independently any integer of 0 or more. In addition, (X)ₘ is located on the N-terminus side and (X)ₙ is located on the C-terminus side. For example, m and n are each independently any integer from 0 to 20, 0 to 15, 0 to 10, or 0 to 5, and the sum of m and n is any integer of 40 or less, 30 or less, 20 or less, or 10 or less.

Next, after identifying the HLA class and/or the type of organ with cancer of a subject such as a patient, a peptide based on a wild-type gene corresponding to a gene having a cancer cell-specific genetic mutation (i.e., "wild-type peptide" described above) is identified from the HLA-presented peptide database such that the HLA class and organ type of the subject match the HLA class and the organ type in the HLA-presented peptide database. In other words, the peptide based on the wild-type gene corresponding to the gene having the cancer cell-specific genetic mutation is identified from the HLA-presented peptide database for each HLA class and each organ type.

More specifically, regarding the peptide having "Wt" and amino acid sequences (i.e., the above-mentioned "(X)ₘ" and "(X)ₙ") at its N-terminus side and C-terminus side which are matched with those of a peptide based on a wild-type gene corresponding to a gene having a cancer cell-specific genetic mutation (i.e., the above-mentioned "wild-type peptide"), whether or not said peptide is registered in the MHC-presented peptide database is examined. If said peptide is registered in the MHC-presented peptide database, this is regarded as a "hit". If said peptide is registered once in the MHC-presented peptide database, this is regarded as "1 hit". For each 1 hit, a maximum of 1 point is added to the score of said peptide. Conversely, if said peptide is not registered in the MHC-presented peptide database, the score for said peptide is 0 points. That is, the peptide based on the wild-type gene corresponding to the gene having the cancer cell-specific genetic mutation is scored based on its HLA presentation frequency in the HLA-presented peptide database.

When the score is greater than 0 points, the peptides registered in the HLA-presented peptide database are listed with their respective amino acid sequences and calculated scores. That is, the above peptide based on the wild-type gene corresponding to the gene having the cancer cell-specific genetic mutation is identified based on the above scoring. At this time, the peptides registered in the HLA-presented peptide database are also given scores between 0 and 1 based on the likelihood of the MS method (i.e., the reliability (FDR) of the amino acid sequence of the HLA-presented peptide), so that scores including decimal points may occur. For example, when a peptide registered in the HLA-presented peptide database is given an FDR of 0.01, 1 point is added to the score of such peptide for each hit, and when a peptide registered in the HLA-presented peptide database is given an FDR of 0.05, 0.5 points are added to the score of such peptide for each hit.

Finally, for the peptides registered in the HLA-presented peptide database, "Wt" is replaced with "Mt". The peptides obtained by such replacement are selected as neoantigens.

### [Characteristics of the neoantigen selection method of the invention]

The characteristics of the neoantigen selection method of the invention are shown in Figure 3. Various embodiments shown in Figure 3 are particular specific examples of the invention and the invention is not limited thereto. In addition, "HLA" may be used interchangeably with "MHC".

According to the neoantigen selection method of the invention, neoantigens derived from a subject can be selected. In particular, according to the neoantigen selection method of the invention, by using a combination of conventional in silico method and MS method, the advantages of high sensitivity and simplicity of the former and high specificity of the latter can be simultaneously obtained. That is, according to the neoantigen selection method of the invention, subject-derived neoantigens can be selected with high sensitivity, high specificity, and simplicity. In other words, the neoantigen selection method of the invention allows for efficient, low-cost, and rapid selection of subject-derived neoantigens. In other words, according to the neoantigen selection method of the invention, subject-derived neoantigens can be selected accurately, easily, and rapidly.

### Example 1. Creation of MHC-presented peptide database (MHC ligandome analysis)

0.5 to 2.0 g of tissues that were rapidly frozen in liquid nitrogen (HLA-A24: 14 cases of human colorectal cancer tissue and 11 cases of human normal large intestine mucosal tissue, HLA-A02: 3 cases of human colorectal cancer tissue and 2 cases of human normal large intestine mucosal tissue, H-2K^{d} and H-2D^{d}: large intestine tissue from 21 5-week-old male and 5 6-week-old female Balb/c mice) were respectively freeze-pulverized in a mixer mill MM400 (Retsch), and then dissolved in 30 mL of lysis buffer (0.25% sodium deoxycholate (Wako), 0.2 mmol/L iodoacetamide (Wako), 1 mmol/L EDTA (Dojindo), 200X protease inhibitor cocktail (Sigma), 1 mmol/L PMSF (Sigma), 1% octyl-b-D glucopyranoside (Dojindo)/DPBS (Gibco)), to prepare a lysate containing MHC-peptide complexes. Such lysate was added to a column with MHC-specific antibodies immobilized thereon to concentrate and purify the MHC-peptide complexes. MHC-presented peptides were eluted from the MHC-peptide complexes using 0.2% trifluoroacetic acid (TFA), and then further concentrated and purified using Sep-Pak tC18 (Waters) and ZipTip U-C18 (Millipore).

The MHC-presented peptides were dissolved in 5% acetonitrile/0.1% TFA and then subjected to nano-LC linked orbitrap mass spectrometry (Easy-nLC 1000 system (Thermo) and Q Exactive Plus (Thermo)) to obtain a peak list. The peptide separation conditions by LC were as follows: 3%-30% solution B (100% ACN/0.1% FA) 80 min gradient, flow rate: 300 nL/min, 3 µm C18 nano HPLC capillary column (75 mm x 20 cm, Nikkyo Technos). The measurement conditions of mass spectrometry were as follows for Full MS, resolution: 70,000 (m/z = 200), AGC target value = 3e6, maximum injection time = 100 ms, and scan range of 350 to 2,000 m/z; and for MS/MS, resolution: 17,500 (m/z = 200), AGC target value = 1e5, maximum injection time = 120 ms. The measurement was performed by Data-dependent MS/MS, Top 10 method, which targets 10 precursor ions with high signal intensity for each scan.

The obtained peak list was collated with the MS reference database (protein sequences obtained from GENCODE, v31 and vM23) using Proteome Discoverer v2.3 (Thermo) software to decipher the amino acid sequences of the MHC-presented peptides. Sequest HT was used for the matching algorithm and Percolator was used for the false positive hit rate (FDR). MHC-presented peptides whose amino acid sequences were deciphered were detected with FDR0.05, and those peptides that were 8-14 amino acids in length were identified. The MHC-presented peptides identified from each sample were integrated to create an MHC-presented peptide database. In the MHC-presented peptide database, the following items are registered: (1) the amino acid sequence of the MHC-presented peptide, (2) the reliability (FDR) of the amino acid sequence of the MHC-presented peptide, (3) the MHC class of the sample from which the amino acid sequence of the MHC-presented peptide is derived, and (4) the type of organ of the sample from which the amino acid sequence of the MHC-presented peptide is derived.

### Example 2. Identification of genes having cancer cell-specific genetic mutations using normal and cancer cells derived from each organ of subject

DNA was extracted from human colorectal cancer tissue, human normal large intestine mucosal tissue, mouse colorectal cancer cell line CT26 (ATCC), and spleen of Balb/c mice (five 6-week-old females) using Allprep DNA/RNA/Protein Kit (Qiagen) or DNeasy Blood & Tissue Kit (Qiagen). Libraries were created using SureSelect Human All Exon V6 (Agilent) or SureSelect Mouse (Agilent), and data was acquired on a NovaSeq 6000 (Illumina) sequencer with 150 bp paired-end reads and an average coverage of 150X (data volume acquired: 18 Gb/sample). The obtained reads were mapped to the reference genome hg38 or mm10 using BWA (v0.7.17, v0.7.10), PCR duplicates were removed using Picard tools (2.18.2, 1.118), and variant calling was performed using GATK (v4.0.5.1, v4), the detected variants were annotated using SnpEff (v4.3t, v4.1), SNPs were filtered using dbSNP and the 1000 Genome Project (www.internationalgenome.org), and genes having cancer-specific genetic mutations were identified by mutually comparing the sequence data of normal cells and cancer cells using Mutect2. Such genetic mutations were SNV, Indel, etc.

### Example 3. Identification of wild-type peptides from MHC-presented peptide database

Among the genes having cancer cell-specific genetic mutations identified in Example 2, for genes having missense mutations, the full-length amino acid sequence and the position of the amino acid mutation of the protein translated from said gene were identified by referring to the CDS base sequence and protein information (GRCh38.p13, GRCm38.p6) registered at NCBI. Next, we examined whether the peptides based on wild-type genes corresponding to said gene were registered in the MHC-presented peptide database created in Example 1. That is, as described above in "Principle of the neoantigen selection method of the invention", peptides based on wild-type genes corresponding to the genes having cancer cell-specific genetic mutations were identified from the MHC-presented peptide database for each MHC class and each organ type. Specifically, the peptides were scored based on their respective MHC presentation frequencies in the MHC-presented peptide database, and identified based on the scoring. Here, when a peptide registered in the MHC-presented peptide database was given an FDR of 0.01, 1 point was added to the score of such peptide for each hit, and when a peptide registered in the MHC-presented peptide database was given an FDR of 0.05, 0.5 points were added to the score of such peptide for each hit.

### Example 4. Selection of neoantigens derived from mouse colorectal cancer cell line

### (1) Selection of neoantigens

As described in Example 3, peptides based on wild-type genes corresponding to the genes having genetic mutations specific to the mouse colorectal cancer cell line CT26 were identified from the Balb/c mouse large intestine tissue-derived MHC-presented peptide database for each MHC class and each organ type. Specifically, the peptides were scored based on their respective MHC presentation frequencies in the MHC-presented peptide database, and identified based on the scoring. Then, amino acid mutations due to the genetic mutations were introduced into the peptides, and the peptides into which the amino acid mutations have been introduced were selected as neoantigens derived from mouse colorectal cancer cell line.

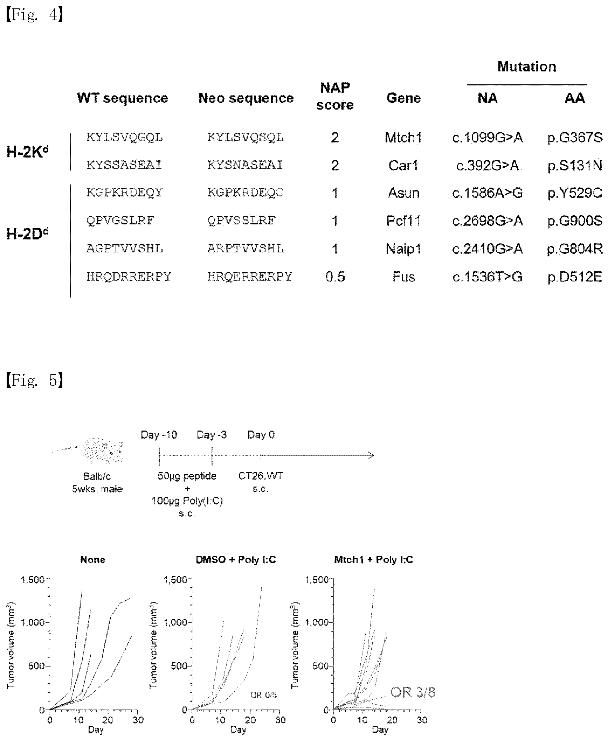

As a result, six neoantigens presented by H-2K^{d} and H-2D^{d}, namely, KYLSVQSQL (SEQ ID NO: 1), KYSNASEAI (SEQ ID NO: 2), KGPKRDEQC (SEQ ID NO: 3), QPVSSLRF (SEQ ID NO: 4), ARPTVVSHL (SEQ ID NO: 5), and HRQERRERPY (SEQ ID NO: 6) (underlined amino acids indicate mutant amino acids) could be selected (Figure 4). The maximum score based on the MHC presentation frequency was 2 points.

### (2) Antitumor effect of selected neoantigens

Peptides were synthesized for each of the six selected neoantigens (Genscript), and the antitumor effect of administration of these peptides to inbred mice were examined. Six-week-old Balb/c mice (male, Hokudo) were subcutaneously administered with a mixture of peptide cocktail containing 50 µg of each of the six neoantigens (or an equal amount of DMSO) and 100 µg of Poly (I:C) HMW (InvivoGen) (adjuvant), 10 days and 3 days before tumor implantation into the mice. A mixture of 5x10⁶ mouse colorectal cancer cell line CT26 and Matrigel (Corning) was subcutaneously implanted in the above mice. Tumors in the mice were measured with a caliper every 2 to 3 days, and tumor volume was calculated using the following formula: Tumor volume = (long diameter of tumor) x (short diameter of tumor)²/2. The control groups (i.e., the untreated group that received no administration and the group that received DMSO and Poly (I:C) HMW (adjuvant)) were conducted with n=5, and the group that received the peptide cocktail and Poly (I:C) HMW (adjuvant) was conducted with n=6.

As a result, in the group administered with the peptide cocktail and Poly (I:C) HMW (adjuvant), a significant antitumor effect was observed in two out of six mice (Figure 5).

Therefore, it was found that an antitumor effect can be obtained by administering neoantigens derived from mouse colorectal cancer cell line selected by the neoantigen selection method of the invention.

### (3) Antitumor effect of combined use of selected neoantigen and anti-PD-1 antibody

Next, the antitumor effect of the combined use of the above peptide cocktail and an immune checkpoint inhibitor (anti-PD-1 antibody) were examined. Four-week-old Balb/c mice (male, Hokudo) were subcutaneously administered with a mixture of peptide cocktail containing 50 µg of each of the six neoantigens and 100 µg of Poly (I:C) HMW (InvivoGen) (adjuvant), 10 days and 3 days before tumor implantation into the mice. The above mice were administered intraperitoneally with 18 mg/kg and 10 mg/kg of anti-mouse PD-1 antibody (clone 29F.1A12, Bio X Cell) 7 and 14 days after tumor implantation, respectively. Tumors in the mice were measured with a caliper every 2 to 3 days, and tumor volume was calculated using the following formula: Tumor volume = (long diameter of tumor) x (short diameter of tumor)²/2. The control group (the group that received anti-PD-1 antibody alone, without administering peptide cocktail and Poly (I:C) HMW (adjuvant)) was conducted with n=9, and the group that received peptide cocktail and Poly (I:C) HMW (adjuvant) and then anti-PD-1 antibody was conducted with n=10.

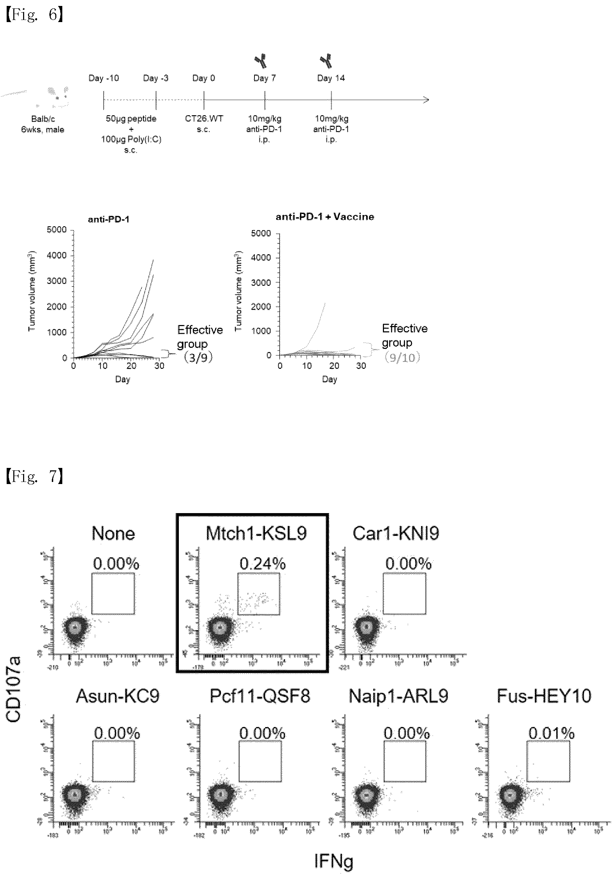

As a result, for the control group (the group that received anti-PD-1 antibody alone, without administering peptide cocktail and Poly (I:C) HMW (adjuvant)), an antitumor effect was observed in 3 out of 9 mice; but for the group that received peptide cocktail and Poly (I:C) HMW (adjuvant) and then anti-PD-1 antibody, an antitumor effect was observed in as many as 7 out of 10 mice (Figure 6).

Therefore, it was found that the neoantigens selected by the neoantigen selection method of the invention can enhance the antitumor effect of immune checkpoint inhibitors.

### (4) Confirmation of induction of CD8-positive T cells for selected neoantigens

In the group that received the foregoing peptide cocktail and Poly (I:C) HMW (adjuvant) followed by anti-PD-1 antibody, the induction of CD8-positive T cells for the peptide cocktail was confirmed using individuals that showed a particularly strong antitumor effect. The spleen was removed from a mouse euthanized by cervical dislocation, and the spleen was passed through a 70-µm cell strainer twice to disperse the cells contained in the spleen. Such cells were then treated with ammonium chloride hemolysis buffer for 5 min to remove red blood cells and collect splenocytes. To 2.5x10⁶ splenocytes, 1 µM of each peptide (each of the six neoantigens listed above), anti-mouse CD16/CD32 antibody (Fc Block, BD), GolgiPlug (BD), Monensin (BioLegend), and anti-mouse CD107a-FITC antibody (BioLegend) were added and incubated at 37°C in a 5% CO₂ environment for 4 h. The splenocytes were then washed with 2% FBS/0.04% NaN₃/PBS, and anti-mouse CD3-PE antibody (BioLenegnd) and anti-mouse CD8-APC antibody (BioLegend) were added to the splenocytes, followed by incubation at 4°C for 20 min. After fixing and permeabilizing the splenocytes with Cytofix/Cytoperm (BD), anti-mouse IFN-γ-PE-Cy7 antibody (BioLegend) was added to the splenocytes, followed by incubation at 4°C for 20 min. Such antibody-stained splenocytes were measured using a flow cytometer (FACS Canto II, BD) and FACSDiva analysis software (BD). Among the cell populations excluding doublets and autofluorescent cells, the CD107a expression level and IFNγ production level of the cell population expressing CD3 and CD8 on the cell surface were measured, and the induction of CD8-positive T cells for each peptide of the six neoantigens was evaluated.

As a result, the induction of CD8-positive T cells was particularly strong for the Mtch1-KSL9 neoantigen, which was detected in 0.24% of the CD8-positive T cell population (Figure 7).

Therefore, CD8-positive T cells are confirmed to be induced for neoantigens selected by the neoantigen selection method of the invention, and it was found that the CD8-positive T cells thus induced bring about an antitumor effect.

### Example 5. Selection of neoantigens derived from human colorectal cancer tissue (CRC) panel

### (1) Selection of neoantigens

As described in Example 3, peptides based on wild-type genes corresponding to the genes having genetic mutations specific to human colorectal cancer tissue (HLA-A24: 15 cases of human colorectal cancer tissue, HLA-A02: 5 cases of human colorectal cancer tissue) were identified from the human large intestine tissue-derived HLA-presented peptide database for each HLA class and each organ type. Specifically, the peptides were scored based on their respective MHC presentation frequencies in the HLA-presented peptide database, and identified based on the scoring. Then, amino acid mutations due to the genetic mutations were introduced into the peptides, and the peptides into which the amino acid mutations have been introduced were selected as neoantigens derived from human colorectal cancer tissue (CRC) panel.

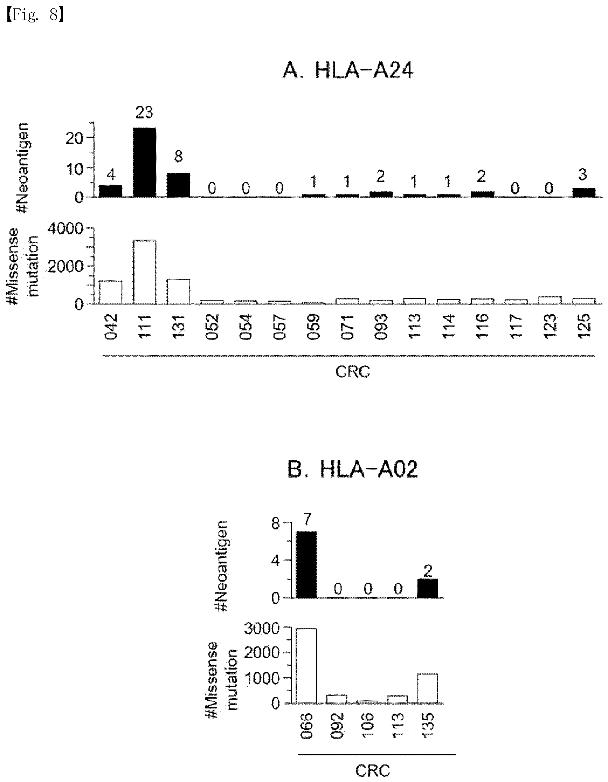

As a result, neoantigens could be selected in several cases of HLA-A24 and HLA-A02 CRC cases (Figure 8). In addition, when the number of missense mutations in patients in each HLA-A24 and HLA-A02 CRC case was examined, the number of selected neoantigens tended to increase in proportion to the number of missense mutations. For example, among HLA-A24 CRC cases, CRC111 case had the largest number of missense mutations in patients, but also the largest number of selected neoantigens.

### (2) Confirmation of TIL response to selected neoantigens

To confirm whether the selected neoantigens actually induce an immune response in the patient's body, tumor-infiltrating lymphocytes (TILs) derived from human colorectal cancer tissue were cultured and examined for TIL response to the selected neoantigens. For this examination, the TUBB-RAF9 neoantigen (RYLAVAAVF (SEQ ID NO: 7)) from CRC111 case (HLA-A24) and the STT3A-KRV9 neoantigen (KLNPQRFEV (SEQ ID NO: 8)) from CRC135 case (HLA-A02) (underlined parts indicate mutant amino acids) were used.

Human colorectal cancer tissue sliced into 1-2 mm pieces was cultured in 10% human serum-added AIM-V medium (Gibco) (1% penicillin/streptomycin, 1% GlutaMAX, 10 mmol/L HEPES, 1 mmol/L sodium pyruvate, 55 mmol/L 2-mercaptoethanol, 2.5 µg/mL amphotericin B, 6,000 U/mL rhIL2) for 2-4 weeks to isolate and grow TILs. The medium was changed every 3 days. TILs were then further grown by adding 30 ng/mL anti-human CD3 antibody (clone OKT3, BioLegend) to the above medium, and TILs were cultured for 2 weeks with PBMCs from healthy individuals who had been irradiated (100 Gy).

1-5x10⁵ TILs, an equal amount of T2 or T2-A24 cells that have been pulsed with 1 µM neoantigen (TUBB-RAF9 neoantigen or STT3A-KRV9 neoantigen) and HLA-presented, Human FcR blocking reagent (MBL), GolgiPlug (BD), Monensin (BioLegend), and anti-human CD107a-PE antibody (BioLegend) were mixed and incubated at 37°C in a 5% CO₂ environment for 4 h. The cells were then washed with 2% FBS/0.04% NaN₃/PBS, and anti-human CD3-PE-Cy7 (BD) and anti-human CD8-APC (BioLegend) were added to the cells and incubated at 4°C for 20 min. After fixing and permeabilizing the cells with Cytofix/Cytoperm (BD), anti-human IFN-γ-FITC antibody (BioLegend) was added to the cells and incubated at 4°C for 20 min. Such antibody-stained cells were measured using a flow cytometer (FACS Canto II, BD) and FACSDiva analysis software (BD). Among the cell populations excluding doublets and autofluorescent cells, the CD107a expression level and IFNγ production level of the cell population expressing CD3 and CD8 on the cell surface were measured, and TIL responses to each of the above two neoantigens were evaluated. To define the non-specific response level of TIL, HIV peptides (HLA-A24: RYLRDQQLL (SEQ ID NO: 9) and HLA-A02: SLYNTVATL (SEQ ID NO: 10)) were used as negative controls.

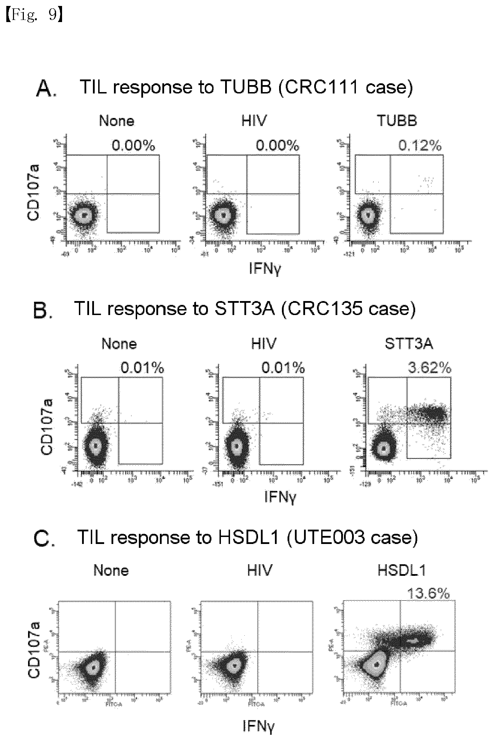

As a result, TIL responses to both of the TUBB-RAF9 neoantigen in CRC111 case (HLA-A24) and the STT3A-KRV9 neoantigen in CRC135 case (HLA-A02) were observed (Figure 9).

Thus, it was found that in human colorectal cancer tissues, CD8-positive T cells that recognize and react to the selected neoantigens infiltrate and accumulate in tumors. That is, it was found that the selected neoantigens actually induce immune responses in the patient's body. In addition, it was found that the neoantigen selection method of the invention can select immunogenic neoantigens that are actually HLA-presented in tumor tissues and serve as an attack target for tumor-infiltrating CD8-positive T cells, with high sensitivity, high specificity, and simplicity.

### (3) Application to HLA class II-presented neoantigens

According to the neoantigen selection method of the invention, not only HLA class I-presented neoantigens but also HLA class II-presented neoantigens can be selected.

As described in Example 3, peptides based on wild-type genes corresponding to the genes having genetic mutations specific to human colorectal cancer tissue were identified from the human large intestine tissue-derived HLA-presented peptide database for each MHC class and each organ type. Specifically, the peptides were scored based on their respective MHC presentation frequencies in the HLA-presented peptide database, and identified based on the scoring. Then, amino acid mutations due to the genetic mutations were introduced into the peptides, and the peptides into which the amino acid mutations have been introduced were selected as neoantigens derived from human colorectal cancer tissue.

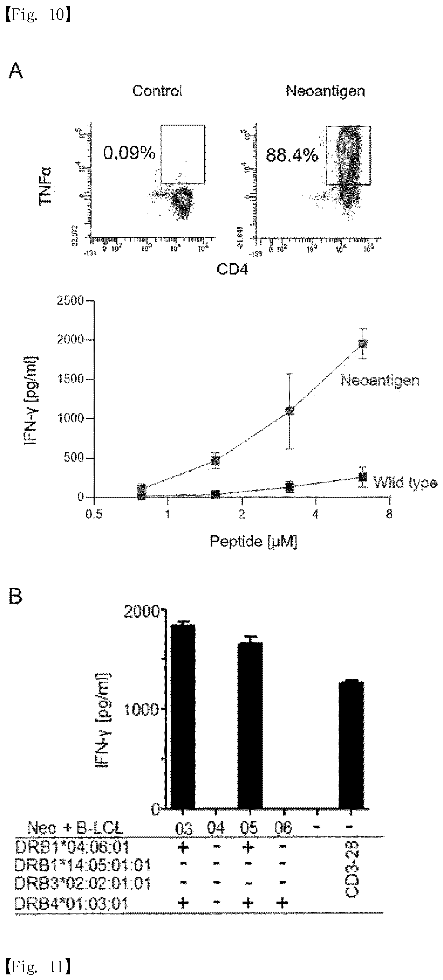

As a result, the SCO1 neoantigen (KGEIAASIVTHMRPY (SEQ ID NO: 11)) (underlined part indicates mutant amino acids) of CRC66 case (HLA-DR*04:06) could be selected (Figure 10). Reactive CD4-positive T cells were cloned from the patient's peripheral blood, and the reaction of the CD4-positive T cell clone with SCO1 neoantigen was examined; as a result, cytokine (TNFα and IFNγ) production specific to SCO1 neoantigen was observed. No such cytokine production was observed for the wild-type peptide corresponding to the SCO1 neoantigen. Furthermore, when antigen-presenting cells of different HLA classes were used for the examination, SCO1 neoantigen presentation on HLA-DR*04:06 could be confirmed.

### Example 6: Effect of combined use of vaccine and immune checkpoint inhibitor (ICB)

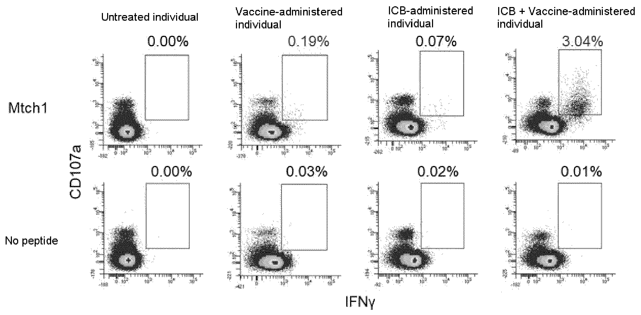

Spleens were collected from CT26 tumor-implanted Balb/c mice that have been untreated, vaccinated, anti-PD-1 antibody-administered, or treated with combined administration of vaccine and anti-PD-1 antibody, respectively, using SEQ ID NO:1 (KYLSVQSQL) as the neoantigen, and the reaction of CD8+ cells to the neoantigen (CD107a expression level and IFNγ production level) was examined; as a result, it was confirmed that combined use of vaccine and immune checkpoint inhibitor (ICB) increased neoantigen-reactive CD8+ cells in the spleen (Figure 11).

### Example 7. Selection of neoantigens in genes identified in Example 2 (using HLA-presented peptides derived from multiple organs)

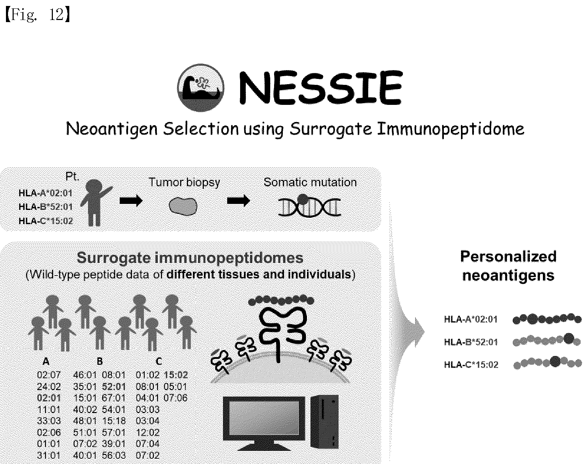

As in Example 1, an MHC-presented peptide database was created from samples containing peripheral blood tissue. Then, as in Example 2, genes having cancer cell-specific genetic mutations were identified. As in Example 3, peptides based on wild-type genes corresponding to genes having tumor-specific genetic mutations, but independent of HLA class and organ type, are identified from a combined database of HLA-presented peptides from various HLA classes and organs. Specifically, the peptides are scored based on their respective MHC presentation frequencies in the HLA-presented peptide database, and identified based on the scoring. Then, amino acid mutations due to the genetic mutations are introduced into the peptides, and the peptides into which the amino acid mutations have been introduced are selected as neoantigens (Figure 12).

### Example 8. Selection of neoantigens in genes identified in Example 2 (using autologous PBMC-derived HLA-presented peptides)

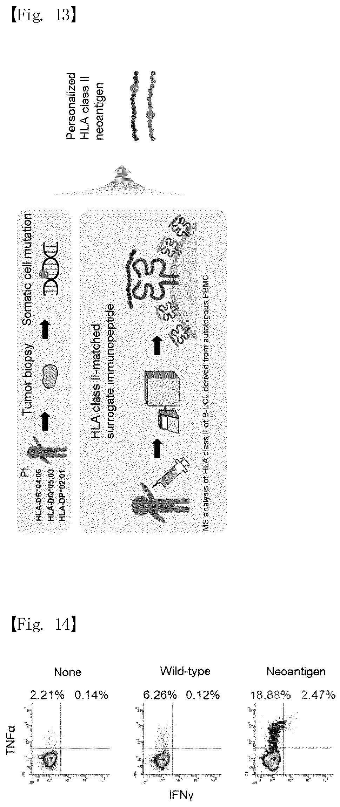

Similar to Example 3, peptides based on wild-type genes corresponding to genes having tumor-specific genetic mutations are identified from an HLA-presented peptide database of B-LCL derived from autologous PBMCs, for neoantigens presented in all HLA classes, especially HLA Class II. Specifically, a database is created from HLA-presented peptidome of B-LCL obtained as in Example 1, and the peptides are identified based on the HLA-presented peptide database. Then, amino acid mutations due to the genetic mutation are introduced into the peptides, and the peptides into which the amino acid mutations have been introduced are selected as neoantigens derived from tumor cells (Figure 13).

As a result, the SCO1 neoantigen (KGEIAASITHMRPY (SEQ ID NO: 11)) (underlined part indicates mutant amino acids) of CRC66 case (HLA-DR*04:06) could be selected (Figure 10). Reactive CD4-positive T cells were cloned from the patient's peripheral blood, and the reaction of the CD4-positive T cell clone with SCO1 neoantigen was examined; as a result, cytokine (TNFα and IFNγ) production specific to SCO1 neoantigen was observed (Figure 14). No such cytokine production was observed for the wild-type peptide corresponding to the SCO1 neoantigen. Furthermore, when antigen-presenting cells of different HLA classes were used for the examination, SCO1 neoantigen presentation on HLA-DR*04:06 could confirmed.

Based on the above results, according to the present invention, neoantigens derived from a subject can be selected. In particular, according to the present invention, by using a combination of conventional in silico method and MS method, the advantages of high sensitivity and simplicity of the former and high specificity of the latter can be simultaneously obtained. That is, according to the present invention, subject-derived neoantigens can be selected with high sensitivity, high specificity, and simplicity. In other words, the present invention allows for efficient, low-cost, and rapid selection of subject-derived neoantigens. In other words, according to the present invention, subject-derived neoantigens can be selected accurately, easily, and rapidly.

Furthermore, according to the present invention, based on the selected subject-derived neoantigen, peptides and/or nucleic acids containing said neoantigen can be produced. In addition, according to the present invention, based on the selected subject-derived neoantigen, peptides and/or nucleic acids containing said neoantigen can be produced, which can be applied to personalized medicine for the subject. Furthermore, according to the present invention, based on the selected subject-derived neoantigen, mRNAs containing said neoantigen can be produced, which can be applied to personalized medicine for the subject.

### [Sequence Listing]

PCT3150SI sequence listing.xml

## Claims

1. A method for selecting a neoantigen derived from a subject, comprising:
(1) a step of acquiring sequence data of a normal cell and a cancer cell derived from the subject,
(2) a step of identifying a gene having a genetic mutation specific to the cancer cell by mutually comparing the sequence data of the normal cell and the cancer cell, and
(3) a step of identifying a peptide based on a wild-type gene corresponding to the gene having the genetic mutation specific to the cancer cell from a major histocompatibility complex (MHC)-presented peptide database, wherein
the genetic mutation specific to the cancer cell is a missense mutation, and
the peptide based on the wild-type gene corresponding to the gene having the genetic mutation specific to the cancer cell has a wild-type amino acid corresponding to the position of an amino acid mutation due to the genetic mutation.

2. The method according to claim 1, wherein the sequence data is whole exome sequence data.

3. The method according to claim 2, wherein the MHC-presented peptide database comprises a list of wild-type MHC-presented peptides for each MHC class and/or each organ type.

4. The method according to claim 1, wherein the MHC-presented peptide database comprises a list of wild-type MHC-presented peptides.

5. The method according to claim 4, wherein step (3) is a step of identifying a peptide based on a wild-type gene corresponding to the gene having the genetic mutation specific to the cancer cell, from the MHC-presented peptide database for each MHC class and/or each organ type.

6. The method according to claim 5, wherein the amino acid mutation due to the genetic mutation is a single amino acid mutation.

7. The method according to claim 6, wherein the peptide based on the wild-type gene corresponding to the gene having the genetic mutation specific to the cancer cell is scored based on its MHC presentation frequency in the MHC-presented peptide database.

8. The method according to claim 7, wherein the peptide based on the wild-type gene corresponding to the gene having the genetic mutation specific to the cancer cell is identified based on the scoring.

9. The method according to claim 8, wherein the MHC-presented peptide database is created by an MHC-presented peptide analysis method comprising a step of obtaining an MHC-peptide complex from a cell, a step of obtaining a peptide from said complex, and a step of identifying the amino acid sequence of said peptide by mass spectrometry.

10. The method according to claim 9, wherein the cell is not from a subject or a non-subject.

11. The method according to claim 9, wherein the cell is collected from blood.

12. The method according to claim 9, wherein the MHC is MHC class I and/or class II.

13. The method according to claim 12, wherein the MHC is a human leukocyte antigen (HLA).

14. The method according to claims 1 to 13, further comprising, in the peptide based on the wild-type gene corresponding to the gene having the genetic mutation specific to the cancer cell, said peptide having a wild-type amino acid corresponding to the position of an amino acid mutation due to the genetic mutation, a step of introducing an amino acid mutation due to the genetic mutation and/or a step of determining the gene sequence encoding the peptide into which the amino acid mutation has been introduced.

15. The method according to claim 14, further comprising a step of selecting the peptide into which the amino acid mutation has been introduced and/or the nucleic acid consisting of the base sequence into which the genetic mutation has been introduced as a neoantigen derived from the subject.

16. A method for producing a peptide containing a neoantigen derived from a subject, comprising a step of adding 0 or more optional amino acids to the N-terminus and/or C-terminus of the neoantigen derived from the subject selected by the method according to claim 15, wherein the number of amino acids contained in the neoantigen to which the amino acids have been added is 40 or less.

17. A peptide obtained by the method according to claim 16.

18. A method for producing a nucleic acid containing a neoantigen derived from a subject, comprising a step of adding 0 or more optional bases to the 5' end and/or 3' end of a nucleic acid containing the neoantigen derived from the subject selected by the method according to claim 15.

19. A nucleic acid containing a neoantigen derived from a subject, wherein the neoantigen is selected by the method according to claim 15.

20. The method according to claim 18, further comprising a step of producing an mRNA containing a nucleic acid containing a neoantigen derived from the subject.

21. An mRNA containing a neoantigen derived from a subject, wherein the neoantigen is selected by the method according to claim 15.

22. A vaccine composition comprising the peptide according to claim 17, the nucleic acid according to claim 19, or the mRNA according to claim 21.

23. A system for selecting a neoantigen derived from a subject, comprising:
(1) a sequence data acquisition section to acquire sequence data of a normal cell and a cancer cell derived from the subject,
(2) a gene identification section to identify a gene having a genetic mutation specific to the cancer cell by mutually comparing the sequence data of the normal cell and the cancer cell, and
(3) a peptide identification section to identify a peptide based on a wild-type gene corresponding to the gene having the genetic mutation specific to the cancer cell from a major histocompatibility complex (MHC)-presented peptide database, wherein
the genetic mutation specific to the cancer cell is a missense mutation, and
the peptide based on the wild-type gene corresponding to the gene having the genetic mutation specific to the cancer cell has a wild-type amino acid corresponding to the position of an amino acid mutation due to the genetic mutation.

24. The system according to claim 23, wherein the cell is not from a subject or a non-subject.

25. The system according to claim 23, wherein the cell is collected from blood.
